# EUROPEAN PATENT SPECIFICATION

(11) **EP 1 649 024 B1**
(45) Date of publication and mention of the grant of the patent: **24.03.2010**
(21) Application number: 04774814.0
(22) Date of filing: 02.07.2004
(51) Int. Cl.: C12N 15/63, C12N 15/74, A23C 19/00, C12R 1/23, C12R 1/46

(54) **METHODS AND MEANS FOR REGULATING GENE EXPRESSION**
VERFAHREN UND MITTEL ZUR REGULATION DER GENEXPRESSION
METHODES ET MOYENS POUR REGULER L'EXPRESSION DES GENES

(30) Priority: 02.07.2003 EP 03077074
(43) Date of publication of application: 26.04.2006
(73) Proprietor: Friesland Brands B.V., 7943 PE Meppel (NL)
(72) Inventor: DEN HENGST, Christiaan, Daniël, NL-9713 VK Groningen (NL); GAJIC, Olivera, NL-6721 CG Bennekom (NL); KUIPERS, Oscar, Paul, NL-9728 XG Groningen (NL); KOK, Jan, NL-9714 HL Groningen (NL); SIKKEMA, Jan, NL-9483 PC Zeegse (NL); GEURTS, Johannes, Marie, Wilhelmus, NL-7559 NK Hengelo (NL); NAUTA, Arjen, NL-6721 CG Bennekom (NL)
(74) Representative: Winckels, Johannes Hubertus F.
(86) International application number: PCT/NL2004/000474
(87) International publication number: WO 2005/003354

(56) References cited:
- WO-A-01/29183
- GUEDON ERIC ET AL: "Pleiotropic transcriptional repressor CodY senses the intracellular pool of branched-chain amino acids in Lactococcus lactis" MOLECULAR MICROBIOLOGY, vol. 40, no. 5, June 2001 (2001-06), pages 1227-1239, XP002267700 ISSN: 0950-382X cited in the application
- SERROR PASCALE ET AL: "CodY is required for nutritional repression of Bacillus subtilis genetic competence" JOURNAL OF BACTERIOLOGY, vol. 178, no. 20, 1996, pages 5910-5915, XP002267701 ISSN: 0021-9193 cited in the application
- SERROR PASCALE ET AL: "Interaction of CodY, a novel Bacillus subtilis DNA-binding protein, with the dpp promoter region" MOLECULAR MICROBIOLOGY, vol. 20, no. 4, 1996, pages 843-852, XP008026640 ISSN: 0950-382X cited in the application
- BERGARA F ET AL: "CodY is a nutritional repressor of flagellar gene expression in Bacillus subtilis." JOURNAL OF BACTERIOLOGY, vol. 185, no. 10, May 2003 (2003-05), pages 3118-3126, XP002267702 ISSN: 0021-9193
- MARUGG JOEY D ET AL: "Identical transcriptional control of the divergently transcribed prtP and prtM genes that are required for proteinase production in Lactococcus lactis SK11" JOURNAL OF BACTERIOLOGY, vol. 178, no. 6, 1996, pages 1525-1531, XP002267703 ISSN: 0021-9193 cited in the application
- RATNAYAKE-LECAMWASAM M ET AL: "BACILLUS SUBTILIS CODY REPRESSES EARLY-STATIONARY-PHASE GENES BY SENSING GTP LEVELS" GENES AND DEVELOPMENT, COLD SPRING HARBOR LABORATORY PRESS, NEW YORK, US, vol. 15, no. 9, 1 May 2001 (2001-05-01), pages 1093-1103, XP001184127 ISSN: 0890-9369 cited in the application
- MOLLE VIRGINIE ET AL: "Additional targets of the Bacillus subtilis global regulator CodY identified by chromatin immunoprecipitation and genome-wide transcript analysis." JOURNAL OF BACTERIOLOGY, vol. 185, no. 6, March 2003 (2003-03), pages 1911-1922, XP002267704 ISSN: 0021-9193 cited in the application
- KONINGS WIL N ET AL: "Lactic acid bacteria: The bugs of the new millennium" CURRENT OPINION IN MICROBIOLOGY, vol. 3, no. 3, June 2000 (2000-06), pages 276-282, XP002315683 ISSN: 1369-5274
- KUIPERS OSCAR P ET AL: "Current strategies for improving food bacteria" RESEARCH IN MICROBIOLOGY, vol. 151, no. 10, December 2000 (2000-12), pages 815-822, XP002315684 ISSN: 0923-2508
- DEN HENGST ET AL: 'The Lactococcus lactis CodY regulon' THE JOURNAL OF BIOLOGICAL CHEMISTRY vol. 280, no. 40, 07 October 2005, pages 34332 - 34342, XP002387018
- LEWIS V. WRAY JR ET AL: 'Expression of Bacillus subtilis ureABC operon is controlled by multiple regulatory factors including CodY, GlnR and Spo0H' JOURNAL OF BACTERIOLOGY vol. 179, no. 17, 01 September 1997, pages 5494 - 5501
- EDA S. ET AL: 'role of the dna sequence downstream of the bacillus subtilis hut promoter in regulation of the hut operon' BIOSCI BIOTECHNOL BIOCHEM vol. 64, no. 3, 2000, pages 484 - 491, XP007906499

## Description

The invention relates to the field of biochemistry, molecular biology and food production. More in particular, the invention relates to methods and means for regulating gene expression. Even more in particular, the invention relates to CodY target sequences.

The Gram-positive lactic acid bacterium *Lactococcus lactis* is an important microorganism in dairy food production. It is part of many starter cultures used in cheese manufacturing, where its function is to degrade the milk protein casein into small peptides and amino acids (Kok and Vos, 1993). *L. lactic,* like other lactic acid bacteria, is a multiple amino acid auxotroph. It has a complex proteolytic system to break down the major milk protein casein into small peptides and free amino acids that are necessary for growth in this medium (Kunji *et al.,* 1996, Christensen *et al.,* 1999). Initial breakdown of casein is carried out by the extracellular cell wall-bound serine proteinase PrtP. Several lactococcal *prtP* genes have been cloned and sequenced (Kok *et al.,* 1985, Kok *et al.,* 1988, de Vos *et al.,* 1989, Kiwaki *et al.,* 1989). Although they are over 98% identical on the amino acid sequence level, the proteinases can have quite different caseinolytic specificities (Visser *et al.,* 1986). For the production of an active proteinase, the product of *prtM,* a gene that is in a back-to-back orientation with *prtP,* is required. The so-called maturase PrtM plays a role as an extracellular chaperone, inducing the pro-proteinase to adopt a conformation in which it is able to autoproteolytically cleave off its pro-region (Kok, 1990, Haandrikman, 1990). Peptides that are produced by the proteinase can be internalized by either one of three different transport systems. Oligopeptides are taken up by Opp, while DtpT and DtpP transport di- and three-peptides respectively (Tynkkynen *et al.,* 1993, Foucaud *et al.,* 1995). Intracellularly, the peptides are further hydrolyzed into smaller peptides and amino acids by the action of over 15 different peptidases (Kunji *et al.,* 1996, Christensen *et al.,* 1999).

Proteinase and maturase production is inhibited in peptide-rich medium (e.g. containing casitone, a tryptic digest of casein) in a number of lactococcal strains (Exterkate, 1985, Laan *et al.,* 1993, Marugg *et al*., 1995, Miladinov *et al.,* 2001). As PrtP expression is not down-regulated in strains that lack the di- and tripeptide transporter DtpT, it was hypothesized that the internal concentration of small (di-tri) peptides, or amino acids derived thereof, are important in the regulation of proteinase production (Marugg *et al.,* 1995). The genetic information for proteinase regulation was shown to be present on a 90-bp subfragment of the *prtP*/*prtM* intergenic region encompassing the transcription start sites of both genes (Marugg *et al.,* 1996). Disruption of an inverted repeat that is present in this region resulted in derepression of the *prtP* and *prtM* promoters in medium with a high peptide concentration.

The expression of genes of other components of the proteolytic system of *L. lactis* is also affected by medium composition. The expression of OppA, DtpT and DtpP is increased when cells are grown in medium with a low peptide concentration (Detmers *et al.,* 1998, Kunji *et al.,* 1996, Foucaud *et al.,* 1995). Moreover, the expression of the peptidases PepX and PepN in *L. lactis* MG1363 was shown to be regulated in a similar way (Meijer *et al.,* 1996). Promoters of *pepC, pepN, pepO1,* and *pepO2* were also reported to be more active in medium with amino acids than in peptide-rich medium (Guedon *et al.,* 2001a). In the same study, the *prtP* promoter was shown to be subject of a similar regulatory circuit.

Recently, a pleiotropic regulator, CodY, has been identified in *L. lactis* MG1363 that represses several genes involved in the processes mentioned above (Guedon *et al*., 2001b). It was investigated what signal controls CodY-dependent expression. It was concluded that branched-chain amino acids (BCAA) are a signal for CodY activity and that CodY is a pleiotropic repressor sensing nutritional supply as a function of the BCAA pool in the cell. CodY, was first identified in the Gram-positive bacterium *Bacillus subtilis,* in which it also serves as a repressor of several genes involved in proteolysis (Serror and Sonenshein, 1996b; Serror and Sonensnein, 1996a).

Serror and Sonenschein (1996b) describe that CodY binds *in vitro* to the dpp promoter region. They consider three general possibilities for the mechanism of action of CodY: (i) CodY binds DNA under the conditions tested, but only represses transcription when activated by an as yet unidentified co-repressor; (ii) CodY is not the direct repressor, but is required to create or maintain a structure that is recognized by the true repressor(s) of CodY-regulated genes; (iii) CodY binds to DNA and recruits the true repressor to the operator site.

Bergara et al. (2003, CodY is a nutritional repressor of flagellar gene expression in Bacillus subtilis, Journal of Bacteriology, vol. 185(10), pages 3118-3126) describe the binding of purified CodY protein to both the *hag* and *fla*/*che* promoter fragments of *Bacillus subtilis.*

Wray et al. (1997, Expression of the Bacillus subtilis ureABC operon is controlled by multiple regulatory factors including CodY, GlnR, TnrA, and Spo0H, Journal of Bacteriology, vol. 179(17), pages 5494-5501) describe that expression of urease in *Bacillus subtilis,* which is encoded by the *ureABC* operon, is regulated by multiple factors including CodY. CodY represses expression of the P2 and P3 promoters of the *ureABC* operon.

Eda et al. (2000, Role of the DNA sequence downstream of the Bacillus subtilis hut promoter in regulation of the hut operon, Biosci. Biotechnol. Biochem., vol 64(3), pages 484-491) concern regulation of the histidine utilization (*hut*) operon in *Bacillus subtilis.* The role of the downstream region of a *hut* promoter in regulation of the *hut* operon was investigated. It was concluded that the position at +14 and probably the position at +23 were involved in CodY-mediated amino acid repression at the *hut* promoter and that CodY was not required for catabolite repression at the *hut* promoter. In B. *subtilis,* the activity of CodY is dependent on intracellular GTP levels, thereby sensing the energy state of the cell (Ratnayake-Lecamwasam *et al.,* 2001). For *L. lactis* it was shown that the

repression by CodY is relieved upon a decrease in the intracellular pool of the branched chain amino acids (BCAA's) Leu, Iso and Val (Guedon *et al.,* 2001b).

Like its *B. subtilis* counterpart, CodY of *L. lactic* contains a C-terminal helix-turn-helix DNA binding motif. In *B. subtilis* it has been shown that the protein is able to bind to sequences overlapping the -35 and -10 sequences of its target promoters (Serror and Sonenshein, 1996b; Fisher, Rohrer, and Ferson, 1996).

Herein we show that CodY represses its target genes by binding to specific DNA sequences upstream of the respective genes. A conserved target site was identified by analyzing upstream sequences of derepressed genes in a delta *codYL. lactis* MG1363 derivative, as identified in a DNA micro-array study. The present application furthermore discloses CodY target sequences from other gram-positive bacteria, like *B. subtilis* and *Streptococcus.*

Hence, the disclosure provides CodY target sequences that may be used in different applications to repress or derepress gene expression.

In a first embodiment, the disclosure provides a method for regulating the expression of a gene of interest in a host cell that comprises a CodY-like protein comprising providing said cell with a gene of interest in operable linkage with a promoter and at least one CodY target sequence.

Regulation of gene expression is a very desirable characteristic of gene expression systems. For example, when one would like to express a protein that is toxic for the used host cell, preferably a gene encoding said protein is under the control of a regulator which can be switched on or off at will. Typically, for production of such a protein, expression of the corresponding gene is suppressed until enough biomass has been obtained and then expression of said gene is obtained for example by providing an inducer. However, also expression of non-toxic proteins is preferably regulated by an induction system. Examples of these kinds of expression systems are well known in the art and hence no further elaboration on this subject is necessary In a method according to the disclosure a CodY-like protein and at least one CodY target sequence, control expression of a gene of interest. Binding of a CodY-like protein to said at least one CodY target sequence results in repression of expression of the gene of interest that is under control of said CodY target sequence. In the absence of (sufficient) CodY-like protein or in the presence of non-functional (i.e. non-binding) CodY-like protein, said gene of interest is expressed. Hence, the invention provides a way for regulating gene expression. As CodY-like proteins are typically found in gram-positive bacteria, for example lactic acid bacteria, the invention preferably provides a method for regulation gene expression in gram-positive bacteria. However, it is clear to the person skilled in the art that necessary components of the method according to the invention, i.e. a CodY-like protein and a CodY target sequence may easily be transferred to for example a gram-negative bacterium.

CodY proteins show a large amount of homology in gram-positive bacteria with a low G+C content. A Blast search with the CodY amino acid sequence of *L. lactis* shows homology with *Bacillus subtilis, Bacillus anthracis, Bacillus halodurans, Bacillus stearothermophilus, Clostridium acetobutylicum, Clostridium difficile, Enterococcus faecalis, Staphylococcus aureus, Streptococcus mutans, Streptococcus pneumoniae* and *Streptococcus pyogenes.* Moreover, these CodY proteins all comprise a DNA binding motif, preferably a helix-turn-helix DNA binding motif. It is furthermore shown that CodY proteins comprise putative GTP binding motifs as summarised in Table 1. Furthermore, binding of these CodY proteins to their target sequences is typically under the influence of the energy level of the cell (GTP) or the intracellular pool of branched chain amino acids or the nutritional value of the medium (nitrogen source like casitone).

A CodY-like protein is typically a CodY protein or a functional equivalent and/or a functional fragment thereof, obtained/derived from a gram-positive bacterium, which CodY protein comprises the above outlined characteristics, i.e. capable of binding to a (consensus) CodY target sequence and sensitive to a change in the energy level of the cell, the intracellular pool of branched amino acids or the medium composition. Examples of CodY proteins are the CodY proteins from *Lactococcus lactis* (Guedon et al, 2001b)) or *Bacillus subtilis* (Serror and Sonenshein, 1996b; Serror and Sonenshein, 1996a). It is clear that for example a CodY protein from *L. lactis* can be modified without significantly changing the above outlined characteristics, for example, by introducing point mutations or (small) deletions. Hence, a CodY-like protein is a Cod protein obtained from a gram-positive bacterium such as *Bacillus subtilis, Bacillus anthracis, Bacillus halodurans, Bacillus stearothermophilus, Clostridium acetobutylicum, Clostridium, difficile, Enterococcus faecalis, Staphylococcus aureus, Streptococcus mutans, Streptococcus pneumoniae, Streptococcus pyogenes,* and *Lactococcus lactis,* possibly comprising mutations which do not interfere significantly with for example the binding of said CodY-like protein to a CodY target sequence and furthermore is sensitive to the energy state of a cell, the intracellular pool of branched amino acids or the medium composition.

The location of the CodY target sequence with regard to the promoter sequence is flexible. The CodY target sequence may be either located upstream, downstream or overlapping with regard to the -35 and -10 sequences. Furthermore, at least one CodY target sequence is used in a method according to the invention. As disclosed herein within the experimental part, an increase in the number of CodY target sequences results in a more pronounced regulation of expression and hence introduction of more than one CodY target sequence is useful depending on, for example, the characteristics of the gene of interest.

The promoter used in a method according to the disclosure is preferably a promoter that is functional in the used host cell. For example, a promoter that is functional in a gram-positive bacterium is used, in operable linkage with a CodY target sequence and a gene of interest, for regulating expression of said gene of interest in a gram-positive bacterium. The prior art provides a large amount of promoter sequences, both from gram-positive as well as gram-negative bacteria, and hence no further elaboration on this item is necessary.

In a preferred embodiment, said promoter and/or said CodY target sequence is heterologous with regard to said gene of interest. In yet another preferred embodiment, said CodY target sequence is heterologous with regard to said promoter. Hence, the invention preferably makes use of combinations in which at least one component (i.e. promoter sequence or gene of interest or CodY target sequence) is different when compared to wild type/natural situation. The gene of interest may be an endogenous gene or a heterologous gene. For an endogenous gene that is already in operable linkage with a promoter, only at least one CodY target sequence has to be introduced (in operable linkage with said promoter and said gene). After introduction of said at least one CodY target sequence, expression of said endogenous gene will, in the presence/absence of CodY-like protein, be repressed/derepressed and hence expression of said endogenous gene is regulated. Furthermore, it is within the scope of the present application to introduce an extra copy of an endogenous gene in operable linkage with its own promoter and/or at least one CodY target sequence or with another promoter and/or at least one CodY target sequence. Hence, at least two copies of said endogenous gene are then present. A heterologous gene in operable linkage with a promoter and at least one CodY sequence may for example be introduced via a plasmid. However, it also possible to only introduce said gene of interest and further provide said gene of interest with the necessary means for homologous recombination to an endogenous gene that is under control of a CodY target sequence. In this way an endogenous gene is replaced by another gene, which is, then under control of a CodY target sequence.

The introduction of new and/or extra genetic information into a host cell may be accomplished by methods known in the art, for example by electroporation, protoplast transformation, transfection, transduction or any other known method.

Any sequence can be used as sequence of interest. Preferably, said sequence enables the production of a protein of interest not present as such or present in a (too) low concentration, in said cell. For example, a sequence/open reading frame (ORF) specifying an enzyme (protease or peptidase), a vitamin or an anti-microbial peptide is used. Preferably, said gene of interest is a gene from a gram-positive bacterium. Even more preferably, said gene of interest is a gene from a lactic acid bacterium, like *Lactococcus, Lactobacillus, Streptococcus, Leuconostoc, Pediococcus, Bifidobacterium, Carnobacterium* or *Propionibacterium.* An example of a gene of interest is a gene that encodes a protease or a peptidase or an anti-microbial peptide or a vitamin. Other examples of gene products include, but is not limited to, hydrolytic enzymes selected from proteases such as chymosin, peptidases including endopeptidases, lipases, nucleases and carbohydrases; lytic enzymes such as lysozyme or phage lysins; flavour enhacing substances; bacteriocins including nisin, pediocin and bavaracin; amino acids; organic acids; and pharmacologically active substances. Further examples comprise genes of which the products make host cells more resistant to advere conditions, for instance conditions to which micro-organisms are confronted at various stages during industrial use, e.g. starvation, lactic acid accumulation, oxygen stress, drying-stress, temperature stress. For example probiotic formula's are made more robust during and/or after production, e.g. by optimising the survival of cells of probiotic strains. As another example, the re-growth of starter cultures is made more reliable.

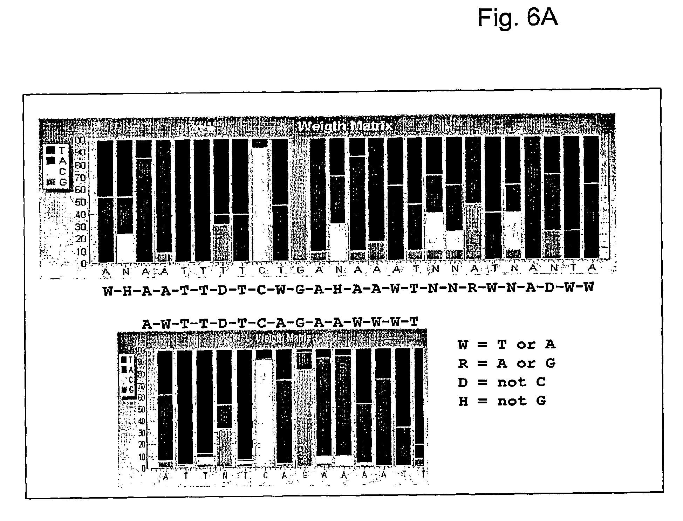

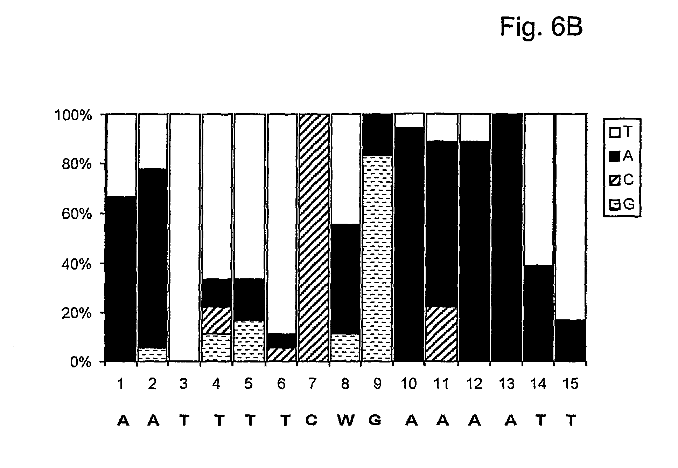

In a preferred embodiment the disclosure provides a method for regulating the expression of a gene of interest in a host cell that comprises a CodY-like protein comprising providing said cell with a gene of interest in operable linkage with a promoter and at least one CodY target sequence, wherein said CodY target sequence comprises a sequence as depicted in the upper part of Figure 6A, or a functional equivalent and/or a functional fragment thereof. The upper part of Figure 6A discloses a consensus CodY target sequence. In another preferred embodiment the disclosure provides a method for regulating the expression of a gene of interest in a host cell that comprises a CodY-like protein comprising providing said cell with a gene of interest in operable linkage with a promoter and at least one CodY target sequence, wherein said CodY target sequence comprises a sequence as depicted in Figure 6B, or a functional equivalent and/or a functional fragment thereof. Moreover, the disclosure provides in Table 4 and Table 4A multiple examples of *L. lactis* CodY target sequences that provide non-limiting examples of combinations of W, R, D and H as depicted in Figures 6A and 6B. Until the present patent application, no (consensus) sequence for CodY binding was disclosed. Now that the consensus sequence and some of its variants are disclosed herein (see upper part of Figure 6A, Table 4 and Table 4A) a person skilled in the art is very well capable of obtaining a functional equivalent and/or a functional fragment of said consensus sequence. A functional equivalent and/or a functional fragment must still be capable of binding a CodY-like protein. A functional equivalent is for example obtained by screening other bacteria for the presence of the herein disclosed CodY target sequences. For example, the present inventors have identified CodY target sequences in *Bacillus subtilis, Streptococcus pneumoniae* and *Streptococcus agalacticiae,* as disclosed herein within Figure 6A lower part, Table 5, 6, 7 or 8. The lower part of Figure 6A discloses the CodY target consensus sequence in *B. subtilis* and Table 5 and 6 show multiple examples of the typical CodY target sequences. Table 7 and 8 disclose multiple examples of *Streptococus* CodY target sequences. It is clear that point mutation and deletion studies lead to further functional equivalents and/or functional fragments and hence these also within the scope of the present patent application.

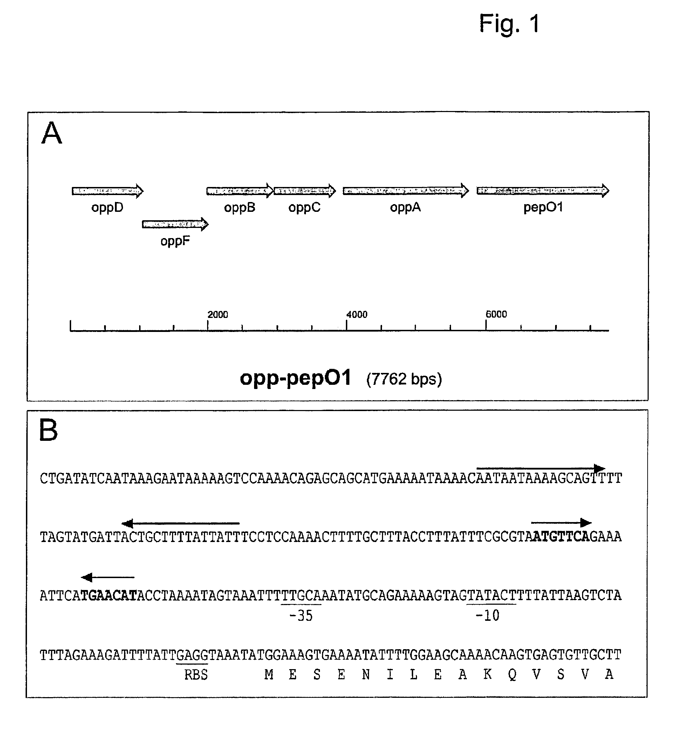

One embodiment of the present disclosure provides a method for regulating the expression of a gene of interest in a host cell that comprises a CodY-like protein comprising providing said cell with a gene of interest in operable linkage with a promoter and at least one CodY target sequence, wherein said CodY target sequence comprises an ATGTTCA sequence and an inversely repeated ATGTTCA sequence. An example of such sequence is shown in Figure 1. As is shown in the examples, said CodY target sequence is involved in CodY-mediated regulation of P*oppD*. Preferably, said nucleic acid sequence comprises a spacing of about 9 base pairs between said ATGTTCA sequence and said inversely repeated ATGTTCA sequence. More preferably, said nucleic acid sequence comprises the sequence
ATGTTCAGAAAATTCATGAACAT.

Based on the herein disclosed (consensus) CodY target sequences it is furthermore possible to construct for example constructs comprising two or more (identical or different) CodY-like target sequences. In this way a more stringent regulation of expression is obtained. For example a gene of interest in operable linkage with a promoter and two (identical or different) CodY target sequence is used to obtain more stringent control of expression. However, it is also possible to introduce a construct that comprises multiple CodY target sequences (with or without a promoter and/or a gene of interest) in a cell that comprises CodY regulated genes and hence a competitive binding of CodY to said construct that comprises multiple CodY target sequences and binding to a CodY target sequence in operable linkage with a gene of interest and a promoter takes place. In this way a gene of interest is derepresses and said gene of interest is expressed.

The method according to the disclosure allows both active as well as inactive/passive regulation of gene expression of a gene of interest. Said regulation is preferably based on influencing the binding between a CodY-like protein and at least one CodY target sequence. An example of passive/indirect/inactive regulation is a gene of interest in operable linkage with a promoter and a CodY target sequence that is introduced into a host cell that comprises CodY-like protein. During exponential growth of said host cell said CodY-like protein binds to said CodY target sequence and hence expression of said gene of interest is repressed. After the exponential phase, said CodY-like protein will release from said CodY target sequence and hence expression of said gene of interest is induced. Such an approach is extremely useful in cases in which one would like to have expression of a gene of interest after exponential growth. Active regulation of gene expression of a gene in operable linkage with a promoter and a at least one CodY target sequence is for example obtained by regulating binding of a CodY-like protein and a CodY target sequence by subjecting said cell to a change in a growth condition, preferably to a growth limiting condition like a limited availability of a nitrogen source. In case, a CodY-like protein or a functional fragment and/or a functional derivative thereof of *L. lactis* is used, means that result in a decrease in the intracellular pool of the branched amino acids Leu, Iso and Val results in relief of CodY repression. The CodY protein of *B. subtilis* is for example actively regulated by a means that influence the level of GTP in a host cell. Hence, actively subjecting a CodY-like protein comprising host cell that further comprises a gene of interest in operable linkage with a promoter and at least one CodY target sequence to a medium with a limited availability of a nitrogen source results in derepression and hence expression of said gene of interest.

In a preferred embodiment, the disclosure provides a method for regulating the expression of a gene of interest in a host cell that comprises a CodY-like protein comprising providing said cell with a gene of interest in operable linkage with a promoter and at least one CodY target sequence, wherein said host cell is a cell from a food production species and even more preferably a dairy food production species. Preferably, said species is selected from the gram-positive species and even more preferably said species is a lactic acid bacterium such as *Lactococcus* or *Lactobacillus* or *Streptococcus* or *Leuconostoc* or *Pediococcus* or *Bifidobacterium* or *Carnobacterium.* An example of a gram-positive, non lactic acid bacterium is *Propionibacterium.* Amongst others, these species are used in the production of food, for example in a fermentation step for the production of a dairy product. Hence, by providing these species with a gene of interest under the control of a promoter and at least one CodY target sequence and either indirectly/passively or directly/actively influencing the binding between a CodY-like protein and its target sequence results in repression or derepression (i.e regulation) of gene expression of said gene of interest. This may be used for the metabolic engineering of various catabolic pathways by a rerouting strategy consisting of the controlled overproduction and/or disruption of genes. For example, genes of which the products, directly or indirectly, are involved in the production of compounds that are involved in the formation of off-flavours during exponential growth during a (dairy) food production, are repressed by providing said genes with a CodY target sequence. Food or dairy food production species in which said genes are under the control of a CodY target sequence, will produce less (or preferably no) off-flavours and hence these production processes are improved. It has for instance become possible to alter flavour formation in cheese, yoghurt and/or other fermented (dairy) products by altering the expression of enzymes that, when present in different quantities, give rise to re-routing of specific pathways. Examples of such enzymes are enzymes involved in e.g.
1) lactose, citrate and diacetyl metabolism, and alcohol metabolism,
2) lipid degradation, modification and synthesis,
3) polysaccharide synthesis,
4) amino acid degradation,
5) protein and carbohydrate utilisation and conversion,
6) cell lysis (e.g. bacteriophage and host cell-encoded cell wall hydrolases and DEAD-box helicase proteins) and cell wall synthesis.

In an analogous way it is also possible to induce expression of a gene of interest after the exponential growth and hence provide said species with altered flavour formation, altered cell lysis capabilities or induce production of antimicrobial substances and/or health promoting substances (such as vitamins) or provide said species with means to at least in part prevent acidification of the same or another species. For the latter possibilities, a gene of interest (for example a gene involved in cell lysis or flavour formation or a gene encoding a vitamin) is placed under the control of a promoter and at least one CodY target sequence and after the end of exponential growth, CodY-like protein will be released from said CodY target sequence and hence expression of said gene is induced. Furthermore, CodY-like proteins are released from their target sequences by providing cells with for example synthetic CodY targets. Said CodY target may be added to the medium, taken up by the cells (for example *B. subtilis)* and the CodY-like proteins are released from their targets and will bind to the synthetic CodY targets. With a method of the invention it has for instance become possible to induce the production of antimicrobial substances (for instance antimicrobial peptides) of which the production would be detrimental during the fermentation process but is benificial after exponential growth to prevent spoilage organisms in the fermented product. A method of the invention is for instance suitable for inducing bacteriocin (e.g. nisin) production in cheese, yoghurt and/or other fermented (dairy) products after exponential growth thereby preventing that the fermenting micro-organisms are affected during fermentation.

In another aspect a method of the disclosure is used in order to decrease the expression of a gene in a stationary phase culture or equivalent of said culture. This is for instance done by providing a host cell with an antisense nucleic acid sequence in operable linkage with a promoter and at least one codY target sequence. In the presence of CodY, expression of said antisense nucleic acid is repressed during exponential growth. Upon transcription of said antisense nucleic acid during the stationary phase, produced RNA will bind mRNA of said undesired gene, thereby preventing translation of said undesired gene. Said undesired gene for instance comprises a gene involved in post-acidification. Said gene for instance comprises a gene involved in carbon catabolism, glucose and lactose catabolism (such as for instance glycolytic enzymes/lactate dehydrogenase) and lactose uptake. It has also become possible to prevent undesired CO₂ production by inhibiting citrate catabolism, to prevent off-flavour production or re-routing of specific pathways by inhibiting expression of a gene encoding undesired proteins/peptides, and/or to extend the shelf life of fermented (dairy) products by decreasing metabolism, ((post-)acidification, glycolysis, lipolysis, proteolysis, peptidolysis) and/or cell lysis.

Hence, with a method of the present invention expression of a gene can be regulated at any stage of growth, for instance during exponential growth and/or during the stationary phase. In a further aspect a method of the invention is used in order to decrease expression of a gene, wherein binding of a CodY-like protein to a CodY target sequence is regulated by subjecting said cell to a change in a growth condition. Preferably, said cell is subjected to a growth limiting condition. In a preferred embodiment said growth limiting condition is a limited availability of a nitrogen source.

Now that the application discloses multiple (consensus) CodY target sequences it is furthermore part of the disclosure to modify endogenous CodY sequences, to for example increase or decrease (or more generally alter) binding of CodY to one of its endogenous target sequences and hence production of an endogenous gene that is under control of the CodY/CodY target sequence regulation may be altered. For example, proteins which expression is under the control of CodY (and hence are not or hardly not produced during the exponential growth phase) but whose product provides advantageous uses when present during exponential growth may now be amended such that binding of CodY to said CodY target sequence is not or hardly not possible under the exponential growth phase conditions. Hence, said protein is expressed during exponential growth and advantage is taken of the properties of said expressed protein.

In case a host cell does not comprise or does not comprise enough CodY-like protein or comprises non-functional (i.e. CodY protein that is not capable of binding to a CodY target sequence) CodY-like protein, the disclosure furthermore provides a method for regulating the expression of a gene of interest in a host cell that comprises a CodY-like protein comprising providing said cell with a gene of interest in operable linkage with a promoter and at least one CodY target sequence wherein said host cell is further provided with a nucleic acid encoding a CodY-like protein. Examples of CodY sequences are already outlined above and hence no further details are provided. In case a host cell does not comprise/express enough CodY-like protein said cell may be provided with either endogenous and/or heterologous CodY-like protein.

In yet another preferred embodiment, the disclosure provides an isolated or recombinant nucleic acid that comprises at least one CodY target sequence or a functional fragment and/or a functional equivalent thereof. It is clear from Figure 6A (lower and upper part), Figure 6B and Tables 4 to 8, that the length of said nucleic acid generally comprises about 15 to 30 nucleotides. A functional fragment and/or a functional equivalent thereof is defined as a fragment and/or equivalent that is capable of binding a CodY-like protein. A functional fragment is for example obtained by introducing an N-, C- or internal deletion. Examples of suitable CodY target sequences are provided herein (Fig. 6A, Fig. 6B and Tables 4 to 8). A nucleic acid that comprises multiple (either identical or different) CodY target sequences is also included herein. Furthermore, artificial or synthetic CodY target sequences are also included herein.

In a preferred embodiment said isolated or recombinant nucleic acid that comprises at least one CodY target sequence or a functional fragment and/or a functional equivalent thereof, further comprises a promoter sequence and/or a promoter sequence in operable linkage with a gene of interest.

In case a host cell does not comprise or does not comprise enough endogenous CodY-like or comprises non-functional CodY protein, the disclosure furthermore provides an isolated or recombinant nucleic acid that comprises at least one CodY target sequence or a functional fragment and/or a functional equivalent thereof wherein said nucleic acid further comprises a gene encoding a CodY-like protein or a functional fragment and/or a functional equivalent thereof.

In a preferred embodiment, said promoter and/or said at least one CodY target sequence is heterologous with regard to said gene of interest and in another preferred embodiment, said CodY target sequence is heterologous with regard to said promoter.

Again, as already outlined above, said gene of interest may either be an endogenous and/or a heterologous gene. Preferably, said gene of interest is a gene from a gram-positive bacterium, such as a gene from a lactic acid bacterium for example *Lactococcus* or *Lactobacillus* or *Streptococcus* or *Leuconostoc* or *Pediococcus* or *Bifidobacterium* or *Carnobacterium.* An example of a gram-positive, non lactic acid bacterium is *Propionibacterium.*

A gene of interest may be any gene, preferably said gene of interest encodes a protease or a peptidase or an anti-microbial peptide or a vitamin. Other suitable examples include hydrolytic enzymes selected from proteases such as chymosin, peptidases including endopeptidases, lipases, nucleases and carbohydrases; lytic enzymes such as lysozyme or phage lysins; flavour enhacing substances; bacteriocins including nisin, pediocin and bavaracin; amino acids; organic acids; and pharmacologically active substances.

In a preferred embodiment, the disclosure provides an isolated or recombinant nucleic acid that comprises at least one CodY target sequence or a functional fragment and/or a functional equivalent thereof, wherein said CodY target sequence comprises a sequence as depicted in Figure 6A and/or Figure 6B, or a functional equivalent and/or a functional fragment thereof. The upper part of Figure 6A, and figure 6B disclose a consensus CodY target sequence. Moreover, the disclosure provides in Table 4 and Table 4A multiple examples of *L. lactis* CodY target sequences that provide non-limiting examples of combinations of W, R, D and H as depicted in Figure 6A and Figure 6B. Until the present patent application, no (consensus) sequence for CodY binding was disclosed.

In a further preferred embodiment, the disclosure provides an isolated or recombinant nucleic acid that comprises at least one CodY target sequence or a functional fragment and/or a functional equivalent thereof, wherein said CodY target sequence comprises a sequence as depicted in Table 4, Table 4A, Table 5, Table 6, Table 7 and/or Table 8, or a functional equivalent and/or a functional fragment thereof.

In another preferred embodiment the disclosure provides an isolated or recombinant nucleic acid that comprises at least one CodY target sequence or a functional fragment and/or a functional equivalent thereof, wherein said CodY target sequence comprises an ATGTTCA sequence and an inversely repeated ATGTTCA sequence. Preferably, said nucleic acid sequence comprises a spacing of about 9 base pairs between said ATGTTCA sequence and said inversely repeated ATGTTCA sequence. More preferably, said nucleic acid sequence comprises the sequence ATGTTCAGAAAATTCATGAACAT.

Now that a consensus sequence and some of its variants are disclosed herein (see Figure 6B, upper part of Figure 6A, Table 4 and Table 4A) a person skilled in the art is very well capable of obtaining a functional equivalent and/or a functional fragment of said consensus sequence. A functional equivalent and/or a functional fragment must still be capable of binding a CodY-like protein. A functional equivalent is for example obtained by screening other bacteria for the presence of the herein disclosed CodY target sequences. For example, the present inventors have identified CodY target sequences in *Bacillus subtilis, Streptococcus pneumoniae* and *Streptococcus agalacticiae,* as disclosed herein within Figure 6A lower part, Table 5, 6, 7 or 8. The lower part of Figure 6A discloses the CodY target consensus sequence in *B. subtilis* and Table 5 and 6 show multiple examples of the typical CodY target sequences. Table 7 and 8 disclose multiple examples of *Streptococus* CodY target sequences. It is clear that point mutation and deletion studies lead to further functional equivalents and/or functional fragments and hence these also within the scope of the present patent application.

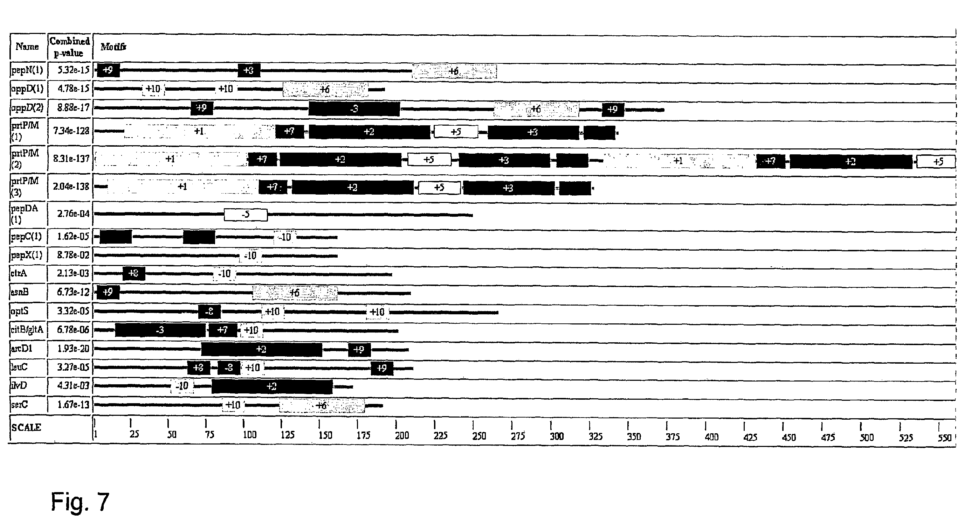

Moreover, the present inventors have identified alternative sequences in the upstream region of CodY regulated genes that may also be included in a method for regulating the expression of a gene of interest in a host cell that comprises a CodY-like protein (Figure 7). These sequences may also be used in a method and/or nucleic acid according to the disclosure.

In another embodiment the disclosure provides a vector comprising a nucleic acid as described above. Said vector may further be provided with means for homologous recombination. With these means said at least one CodY target sequence and/or a gene of interest and/or a promoter and/or a gene encoding CodY-like protein may be integrated into the genome of a cell and hence a more stable situation may be obtained. In yet another embodiment, the disclosure provides a gene delivery vehicle comprising a nucleic acid or a vector according to the invention. Gene delivery vehicles are well known in the art and hence no further details are provided on this subject matter.

In a further embodiment the disclosure provides a host cell that comprises a nucleic acid, a vector or a gene delivery vehicle according to the disclosure. Preferably, said host cell is a cell from a food production species and even more preferably said host cell is a cell from a dairy food production species. Non-limiting examples of said species are gram positive lactic acid bacteria such as *Lactococcus* or *Lactobacillus* or *Streptococcus* or *Leuconostoc* or *Pediococcus* or *Bifidobacterium* or *Carnobacterium.* An example of a gram positive, non lactic acid species is *Propionibacterium.*

In another embodiment, the disclosure provides use of at least one CodY target sequence for regulating the expression of a gene of interest. Preferably, said at least one CodY target sequence is selected from Figure 6A. Figure 6B or Tables 4 to 8. For example, the use of at least one CodY target sequence in operable linkage with a promoter and a gene of interest in a Cod-like protein comprising host cell result, for example under exponential growth in repression of expression of said gene of interest. After exponential growth, CodY protein will be released from its target, resulting in depression of expression of said gene of interest.

In yet another embodiment the disclosure provides a method for producing a food product comprising a nucleic acid or a vector or a gene delivery vehicle or a host cell as described above. Preferably said food product is a dairy food product. As a non-limiting example, the use of a host cell is described in more detail. A lot of (dairy) food production processes involve the use of a (fermenting) host cell. These host cells may now be manipulated with regard their protein products. For example, genes of which the products, directly or indirectly, are involved in the production of compounds that are involved in the formation of off-flavours during exponential growth during a (dairy) food production, are repressed by providing said genes with a CodY target sequence. Food or dairy food production species in which said genes are under the control of a CodY target sequence, will produce less (or preferably no (detectable)) off-flavours during exponential growth and hence these production processes are altered. In an analogous way it is also possible to induce expression of a gene of interest after the exponential growth of said host cell and hence provide said species with altered flavour formation, altered cell lysis capabilities or induce production of antimicrobial substances and/or health promoting substances (such as vitamins) or provide said species with means to prevent acidification of the same or another species. For the latter possibilities, a gene of interest (for example a gene involved in cell lysis or a gene involved in flavour formation or a gene encoding a vitamin) is placed under the control of a promoter and at least one CodY target sequence and after the end of exponential growth, CodY-like protein will be released from said CodY target sequence and hence expression of said gene is induced.

Furthermore expression of undesired genes during the stationary phase can be, at least in part, decreased.

With regard to a fluid dairy product a method to at least in part decrease lysis of bacteria and/or acidification after production of said fluid diary product, i.e. after exponential growth of the used bacteria, is very advantageous with regard to the shelf life of said fluid dairy product. First, genes that are capable of at least in part preventing lysis and/or acidification are identified. After identification, such genes are placed under the regulation of at least one CodY target sequence. Said genes are expressed and hence lysis and/or acidification is at least in part prevented (and more preferably completely inhibited) and hence the shelf life of said product is increased.

Moreover, in case integration of said nucleic acid or vector is desired, use may be made of food grade integration techniques (for example see Leenhouts, 1995, herein incorporated by reference).

Preferably, the disclosure provides a method for producing a (dairy) food product comprising a nucleic acid or a vector or a gene delivery vehicle or a host cell as described herein, wherein said dairy product is a cheese or a fermented milk product. The production of a lot of dairy products involves fermentation of lactic acid bacteria and hence the application of modified host cells as described herein are particularly advantageous. The term "dairy product" include but is not limited to cheese, fluid dairy products like milk and yoghurt, fermented milk product, ice cream, butter, buttermilk, margarine and milk powder.

In another embodiment the disclosure provides food or a dairy food, such as a cheese or a fermented milk product, obtainable by a method according to the disclosure. Such a product comprises for example a different or extra taste or comprises specific compounds/structures (such as health improving compounds as vitamins) not present in food or dairy food product not obtained according to a method of the disclosure. Food or dairy food products prepared by a method according to the invention may comprise different amounts or different kinds of enzymes, peptides, amino acids, flavour enhancing or pharmacologically active substances or organic acids.

In yet another embodiment, the disclosure provides a method for at least in part preventing the formation of off-flavours during a process for the production of a (dairy) food product, comprising providing at least one CodY target sequence in operable linkage with a gene which product is, directly or indirectly, involved in the formation of off-flavours.

Now that the present inventors have disclosed CodY target sequences, interesting leads are provided in the fight against food-spoilers and pathogens.

PpmA is a recently identified pneumococcal protein with significant sequence homology to the proteinase maturation protein (PrtM) of lactic acid bacteria. PrtM is a trans-acting protein involved in the processing of precursors of serine protease PrtP into active enzymes and belongs to the family of peptidyl-prolyl cis/trans isomerases. These enzymes are thought to assist in protein folding by catalyzing the *cis*/*trans* isomerization of the petidylprolyl bonds in peptides and proteins. The pneumococcal proteins(s) that is activated by PpmA is currently unknown. PpmA of *Streptococcus pneumoniae* was demonstrated to be involved in virulence. Inactivation of *ppmA* significantly reduced the virulence of strain D39 for mice as judged by the survival time after intranasal challenge. The present inventors identified a CodY target sequence upstream of *ppmA* (Table 7) indicating that the expression of this gene probably is under the control of CodY. Hence, the invention provides a method for regulating the expression of *ppmA.*

Spore-forming bacteria (e.g. *Bacillus)* can cause serious problems in industrial food fermentations as the spores can survive most processing conditions. In *Bacillus subtilis* it was shown that the target genes of CodY generally encode proteins useful to the cell in adapting to poor nutritional conditions, but also include several genes whose expression is critical to the acquisition of genetic competence and the initiation of sporulation. The present disclosure provides a method for influencing the expression of these genes.

In *Clostridium difficile,* the synthesis of two toxin proteins responsible for antibiotic-associated colitis and pseudomembranous colitis were shown to be dependent on a RNA polymerase sigma factor TxeR (Sonenshein, unpublished). Both TxeR and the toxin proteins were not synthesized in exponential phase cells, probably due to the action of CodY as it was shown to bind to the toxin regulatory region.

The invention will now be illustrated by means of the following, non-limiting examples.

### EXPERIMENTAL PART

### MATERIALS AND METHODS

### Bacterial strains, plasmids and growth conditions

The strains and plasmids used in this study are listed in Table 9. *Escherichia coli* was grown in TY medium at 37°C with vigorous agitation or on TY medium solidified with 1.5% agar, containing 100 µg of erythromycin per ml when needed. 5-bromo-4-chloro-3-indolyl-β-D-galactopyranoside (X-gal) was used at a final concentration of 40 µg/ml. *L. lactis* was grown at 30°C in M17 broth or on M17 medium solidified with 1.5% agar, supplemented with 0.5% glucose. When needed, erythromycin, chloramphenicol and X-gal were added at final concentrations of 5 µg, 5 µg and 80 µg per ml, respectively. Chemically defined medium (CDM) was prepared according to Poolman and Koning (1988).

### DNA preparation, molecular cloning and transformation

Routine DNA manipulations were performed as described by Sambrook *et al*.(1989). Total chromosomal DNA from *L. lactis* MG1363 was extracted as described previously. Plasmid DNA was isolated by the alkaline lysis procedure described by Sambrook *et al* (1989). Minipreparations of plasmid DNA from *E. coli* and *L. lactis* were made using the High Pure Plasmid isolation Kit from Roche, with minor modifications for *L. lactis.* Restriction enzymes and T4 DNA ligase were purchased from Roche. PCR amplifications were carried out using *Pwo* DNA polymerase for cloning fragments and *Taq* DNA polymerase for checking DNA insert sizes in plasmids from transformants. Electrotransformation of *E. coli* and *L. lactis* were performed with a Bio-Rad Gene Pulser (Bio-Rad Laboratories, Richmond, Calif.).

### RNA preparation and primer extension

The *opp* transcript was subjected to primer extension analysis using the oligonucleotide stol4 (CTTGCCATGGAATCACCCG) essentially as described previously (Buist et al, 1997). In the reactions, 30 µg of total RNA that was isolated from *L. lactis* MG1363 cells as described (van Asseldonk et al, 1993) was used as template. A DNA sequence ladder was obtained using the T7 sequencing kit (Amersham Pharmacia Biotech, Little Chalfont, United Kingdom) according to the manufacturers' descriptions.

### Cloning of oppD promoter fragments

In order to study the regulation of promoter strength upstream of the *oppD* a deletion analysis of the respective promoters was carried out. Combinations of oligonucleotides opp1 (5'
GCTCTAGACACTCACTTGTTTTGCTTCC 3')'
opp2 (5' AACTGCAGGAAAATTCATGAACATACC 3'),
opp1-opp3 (5' AACTGCAGTAAAACAATAATAAAAGCAG 3'),
opp1-opp4 (5' AACTGCAGGATAATAAAATTTGGACTG 3'),
opp1-opp14 (5' AACTGCAGCGTAATGTTCAGAA.AATTC 3'),
opp1-opp15 (a) (5' AACTGCAGCGTAATATTTAGAAAATTCATGAACATACC 3') and
opp1-opp15 (b) (5' AACTGCAGCGTACTGTGCCGAAAATTCATGAACATACC 3') were used to amplify chromosomal DNA from *L. lactis* MG1363 in order to obtain fragments encompassing the *oppD* promoter. The PCR products were digested with *Xba*I and *Pst*I and transcriptionally fused upstream of the promoterless *lacZ* gene in the integration vector pORI13 (Sanders *et al.,* 1998), also digested with the same enzymes. The resulting plasmids were called pORIopp2, pORIopp3, pORIopp4, pORIoppl4, pORIopp15 (a) and pORIopp15 (b). All pORI constructions were preformed in *E*. *coli* EC101 which contains a chromosomal copy of the lactococcal *repA* gene needed for replication. They were then transformed into *L. lactis* LL108 and/or LL302 which contain multiple and single chromosomal copies of *repA,* respectively.

### Random mutagenesis of oppD promoter region

PCR fragments encompassing the *oppD* promoter region containing randomly introduced base pair substitutions were obtained essentially as described (Spee, de Vos, and Kuipers, 1993). Chromosomal DNA isolated from *L. lactis* MG1363 cultures were used as a template in the amplification step. Subsequently, the obtained variants were cloned into plasmid pORI13 and introduced into *L. lactis* LL108 as described above. Mutants showing distorted blue coloring on plates containing X-gal were selected and analyzed in more detail as described in results.

### Construction of a codY deletion strain.

A 1400 bp *Eco*RI/*Hin*DIII chromosomal fragment of *L. lactis* MG1363, containing *codY,* was subcloned in pUC19. The resulting plasmid was digested with *Sna*BI and subsequently selfligated. In this way, 423 bp were deleted from *codY.* The oligonucleotides cod280A (5' GGGAATTCGGATTGTCTATCTGCCTCG 3') and cod280B (5' GGGGGATCCAGATCTGACCATGATTACGCCAAGCTT 3') were used to amplify the Δ*codY*-containing fragment. PCR product was digested with *Eco*RI/*Bam*HI (restriction sites are underlined in the oligonucleotide sequence) and ligated into corresponding sites in pORI280. The resulting plasmid, pORIΔ*codY*, was introduced together with pVE6007 into *L. lactis* MG1363. As this strain does not contain the *repA* gene, selection for growth in the presence of erythromycin and increased temperature (37°C) forces pORIA*codY* to integrate into the chromosome by homologous recombination. A number of integrants were subsequently grown for about 30 generations under nonselective conditions allowing a second recombination event to occur, which results in either the deletion or the wild-type gene *codY.* The *ΔcodY* mutation was confirmed by PCR.

### β-Galactosidase activity assay

*In vivo* β-galactosidase (β-gal) assays were carried out in a Tecan microplate reader. Overnight cultures of *L. lactis* grown in GM17 were washed twice in 0.9% NaCl before inoculation to 2.5% in 200µl of the appropriate medium containing erythromycin (5µg/ml) for maintenance of pORI13 in *L*. *lactis* LL108/LL302 and erythromycin and chloramphenicol (2.5µg/ml each) (Leenhouts *et al.,* 1996). The media also contained the β-gal substrate 2% β-trifluoromethylumbelliferyl β-D-galactopyranoside (Molecular probe T-657). Multilabelling experimental data (absorbance and fluorescence measurements) were processed using the Magellan software program. β-Gal production due to the transcription driven from the *oppD* upstream region was calculated as a function of light emission. β-Gal assays were performed throughout the growth of *L. lactis* grown in media in which growth rates differ significantly as a function of the nitrogen source i.e. CDM 0.2% casitone and CDM 2% casitone and measured of the culture. β-Galactosidase activities were determined in permeabilized cell suspensions as described previously (Israelsen et al, 1995). β-Galactosidase enzyme activities, calculated as an average of three independent experiments, were expressed in arbitrary units (Miller, 1972).

### Overproduction and purification of His₆-CodY.

The chromosomaly located *codY* of *L. lactis* MG1363 was amplified by PCR with the oligonucleotides HC-5 (5' CTAGACCACCATGGGG CATCACCATCACCATCACGTGGCTACATTACTTGAAAAAACACG 3'), introducing the underlined *Nco*I restriction enzyme site upstream of the hexa-histidine tag (italic) and
HC-6 (5' CTAGTCTAGATTAGAAATTACGTCCAGCAAGTTTATC 3'),
introducing the underlined *Xba*I restriction enzyme site downstream of the stop codon (italic) of *codY.* The purified 833-bp PCR product was digested with *Nco*I and *Xba*I and ligated into the corresponding sites of pNZ8048, downstream of the nisin-inducible P_{nisA}. The resulting plasmid, pNH6CodY, was introduced in *L. lactis* NZ9000 to enable nisin induction of *his6-codY,* as described (de Ruyter, Kuipers, and de Vos, 1996;Kuipers *et al.,* 1998). His6-CodY was isolated by affinity chromatography in an FPLC procedure (Amersham Pharmacia Biotech) using Ni-NTA agarose (Qiagen GmbH, Hilden, Germany).

### Electrophoretic mobility shift assays (EMSA's)

Gel retardation experiments were carried out essentially as described by Ebbole and Zalkin (Ebbole *et al.,* 1989). Purified PCR products (2 µg) were end-labelled with polynucleotide kinase (Amersham Pharmacia Biotech) for 1 h at 37°C using 30 µCi [γ-³²P]-ATP (Amersham Pharmacia Biotech) in a volume of 20 µl. Reactions were stopped by incubating the mixtures for 10 min at 70°C. Binding studies were carried out in 20 µl reaction volumes containing 20 mM Tris-HCl (pH 8.0), 8.7 % (v/v) glycerol, 1 mM EDTA (pH 8.0), 5 mM MgCl₂, 100 mM KCL, 0.5 mM DTT, labelled DNA fragment (3000 cpm), and purified His6-CodY protein (50-400 ng). BSA (1 µg) and poly(dI-dC) (Amersham Pharmacia Biotech) were added to the reaction mixtures in order to reduce non-specific interactions. After incubation for 15 min at 30°C, samples were loaded onto a 4% polyacrylamide gel. Electrophoresis was performed in the Protean II Minigel System (Bio Rad Laboratories B.V., Veenendaal, The Netherlands) using a gradient (0.5x to 2x) of TAE buffer (Sambrook, Fritsch, and Maniatis, 1989) at 150 V for 1.5 h. Gels were dried and used for autoradiography at - 80°C using Kodak XAR-5 films and intensifying screens.

### Preparation of cells for transcriptome analysis

Cells were grown at 30°C in GM17 supplemented with 0.5% glucose. Cells were grown till mid-exponential phase (OD600∼1.0). Approximately 5x10⁹ cells (50 ml culture) were harvested by centrifugation for 5 min at 10.000 rpm and 4°C. Cells were resuspended in 2 ml ice-cold growth media and divided over 4 screw-cap tubes with rubber seal. After the addition of 500 µl Phenol/Chloroform, 30 µl 10% SDS, 30 µl 3 M NaAc (pH5.2) and 500 mg glass-beads (diameter 75-150 µm), cells were frozen in liquid nitrogen and stored at -80°C or immediately used for RNA isolation.

### Transcriptome analysis

The DNA micro array experiments were essentially performed as described earlier (Kuipers *et al.,* 2002), with the following modifications.

### RNA isolation

For each RNA isolation, one aliquot of the stored cell samples was used. Cells were disrupted by mechanical force using the Savant FastPrep FP120 system (Omnilabo) for 40 seconds at setting 5.0. Subsequently, RNA was extracted using the Roche "High Pure RNA Isolation Kit" according to the provided protocol. RNA yield and quality were determined spectrophotometrically and by performing a RNA 6000 Nano Labchip assay (Caliper Inc.) on the Agilent 2100 Bioanalyzer (Agilent Technologies, Amstelveen, the Netherlands) according to the manufacturers description respectively.

### cDNA labeling

Single-strand reverse transcription (amplification) and indirect labeling of 25 µg of isolated total RNA with either Cy3- or Cy5-dye were done with the Amersham CyScribe Post Labelling Kit according to the manufacturers protocol and, subsequently, used for hybridization.

### Hybridisation and scanning

Sylilated slides (Cel Associates) on which 2145 amplicons of *L. lactis* strain IL1403 were spotted in duplicate were used in the hybridization procedure. In addition, the slides contained 96 amplicons from *L. lactis* strain MG1363. Slides were pre-hybridized in Ambion SlideHyb buffer for 15 min at 40°C in a Genomic Solutions Hybstation. After removal of the pre-hybridization buffer, 10-25 µl of the Cy3/Cy5-labeled cDNA mix in 150 µl Ambion SlideHyb buffer I was added and incubated for 1 h at 42°C and finally for 16 h at 40°C. Afterwards, the hybridized slides were washed for 1 min in 2x SSC, 0.5% SDS and 5 min in 1×SSC, 0.25% SDS. The slides were scanned using a confocal laser scanner GeneTAC LS IV).

### Signal analysis

After scanning of the slides with the GeneTAC LS IV, individual spot intensities were determined. The raw data, along with the scanning image were stored in the Molecular Genetics Information System (MolGenIS). A grid definition was made to enable the spot analysis software Array Pro (Phoretix) to produce tables containing gene names and signal intensities. Using the program Excel (Microsoft corporation), signal intensities were corrected for background and the ratios in signal intensity between the different samples were determined.

### DNaseI footprinting analysis

DNaseI footprinting was performed essentially according to the description supplied with the Sure Track Footprinting Kit (Amersham Pharmacia Biotech). The DNA fragments were prepared by PCR, using Expand DNA polymerase (Amersham Pharmacia Biotech) and the oligonucleotides opp1 and opp3, one of which was first end-labeled with T4 polynucleotide kinase (Amersham Pharmacia Biotech) and [Y-³²P]ATP as described by the manufacturer.

Binding reactions were identical to those used in EMSAs, in a total volume of 40 µl and in the presence of approximately 150,000 cpm of DNA probe. DNAseI footprinting experiments were then performed as described previously (Hamoen et al, 1998)

### In vitro protein cross-linking 5

Cross-linking reactions were performed in 1x cross-linking buffer (100 mM KCl, 15 mM Tris- HCl pH 7.5) in a total volume of 40 µl, containing 300 ng of purified H6-CodY, and, where indicated, GMP (1 mM) or GTP (1 mM). Formaldehyde was used as the cross-linking agent at a final concentration of 1% (v/v), with a 10 min incubation step at room temperature. Cross-linking reactions were stopped by the addition of 20 µl of 2x SDS gel loading buffer [4% SDS, 10% 2-mercaptoethanol, 20% glycerol, 100 mM Tris-HCl (pH 6.0)]. Samples were boiled for 5 min before separation by SDS polyacrylamide (10%) gel electrophoresis (Laemmli, 1970). SDS polyacrylamide gels were stained with Coomassie Brilliant Blue (Bio-Rad).

### EXPERIMENTAL PART

### RESULTS

### Determination of the transcription start site of oppD.

It has been demonstrated that the genes of the oligopeptide permease system, encoded in the *oppDFBCA-pepO1* locus of *L. lactis* MG1363, are transcribed polycistronically. Upstream of both *oppD* and *oppA* regions are present that could serve as promoter elements. To determine the location of the promoter upstream of *oppD,* the transcription start site (TSS) was determined. The *opp* transcript was analyzed by primer extension, using RNA that was isolated from exponentially growing MG1363 cells. The mRNA 5' end corresponds to an adenine residue located 35 bases upstream of the translation start codon AUG of *oppD.* The -35 sequence (TTGCAA) is separated by a consensus 17 bases from the -10 region (TATACT) and a proper lactococcal ribosome binding site; GAGG is also present. The sequence upstream of *oppD* contains two regions of dyad symmetry centered around position -135 (-14.0 kcal/mol) and -62 (-5.6 kcal/mol) relative to the *oppD* transcriptional start site, respectively.

### CodY specifically binds to the oppD upstream region.

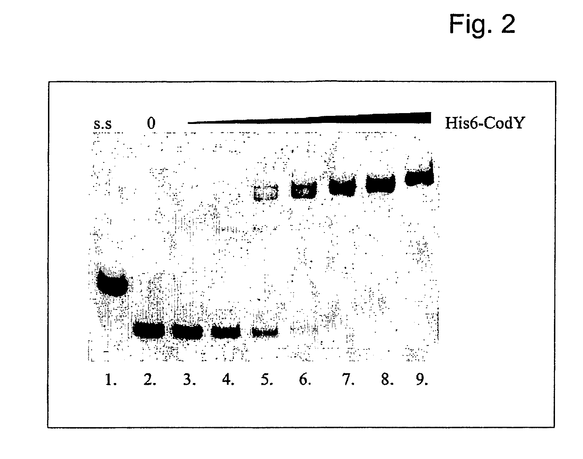

In order to examine whether *L. lactis* CodY directly interacts with upstream DNA sequences of its main target known so far, *oppD, in vitro* DNA binding studies were performed. For this purpose, histidine-tagged CodY was overexpressed using the nisin inducible gene expression system (Kuipers *et al.,* 1998) and subsequently purified to apparent homogeneity. A radioactively labeled PCR fragment spanning the *oppD* upstream region (Fig. 1) was used as a probe. The electrophoretic mobility shift assays (EMSA's) clearly showed that purified H6-CodY is capable of binding directly to a region encompassing the *oppD* promoter (Fig. 2). Multiple retarded fragments were observed, indicating that, probably, multimerization of H6-CodY on the *oppD* promoter occurs.

Upon gradually increasing the amount of H6-CodY from 10 to 100 ng, four distinct bands can be observed, indicating a tetrameric state of the protein. In most of our DNA binding experiments, a band corresponding to single stranded probe (s.s. DNA) was observed irrespective of the presence of H6-CodY. The occurrence of this denatured DNA probably results from the high AT content of the *opp* promoter region. No H6-CodY binding occurred when DNA fragments, with similar AT contents, were used that were obtained from internal gene segments (*e.g.* from *comG of B. subtilis),* indicating that H6-CodY binding to *PoppD* is specific (data not shown).

### CodY regulates the expression of oppD by binding to a specific upstream sequence.

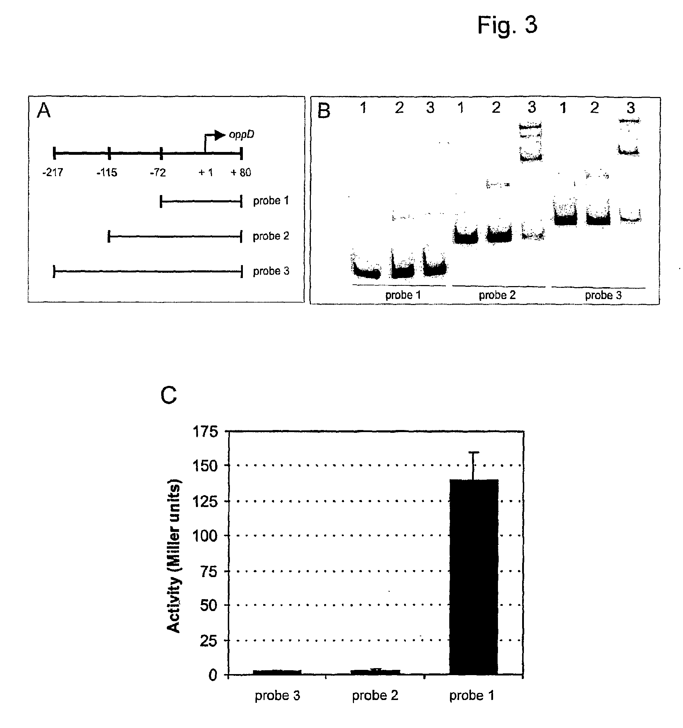

The upstream *oppD* region contains a small sequence that is inversely repeated with a spacing of 9 base pairs in between (Fig. 1). This region of dyad symmetry is located just upstream of the -35 promoter sequence. To determine whether this region is important for the interaction with CodY, a stepwise deletion analysis of the *oppD* upstream region was performed. Radioactively labeled deletion fragments of the region, obtained by PCR, were incubated with H6-CodY. As shown in Fig. 3, the fragments with an extended deletion of part of the *oppD* upstream region showed altered binding of CodY. Two truncated fragments, shortened from the 5'-end of the *oppD* upstream region contained in *opp162,* were obtained by PCR and examined for H6-CodY binding (Fig. 3A and 3B). Probe 2, spanning the region from -111 to +75, was still bound by H6-CodY, suggesting that nucleotides that are critical for CodY binding must reside downstream of the 5'-end of this probe. When a 160-bp DNA fragment was used, of which the 5'-end coincides with the center of the inverted repeated closest to the RBS of *oppD* (Fig. 1B), no binding of H6-CodY was observed, irrespective of the amount of protein added (probe 1 in Fig. 3B).

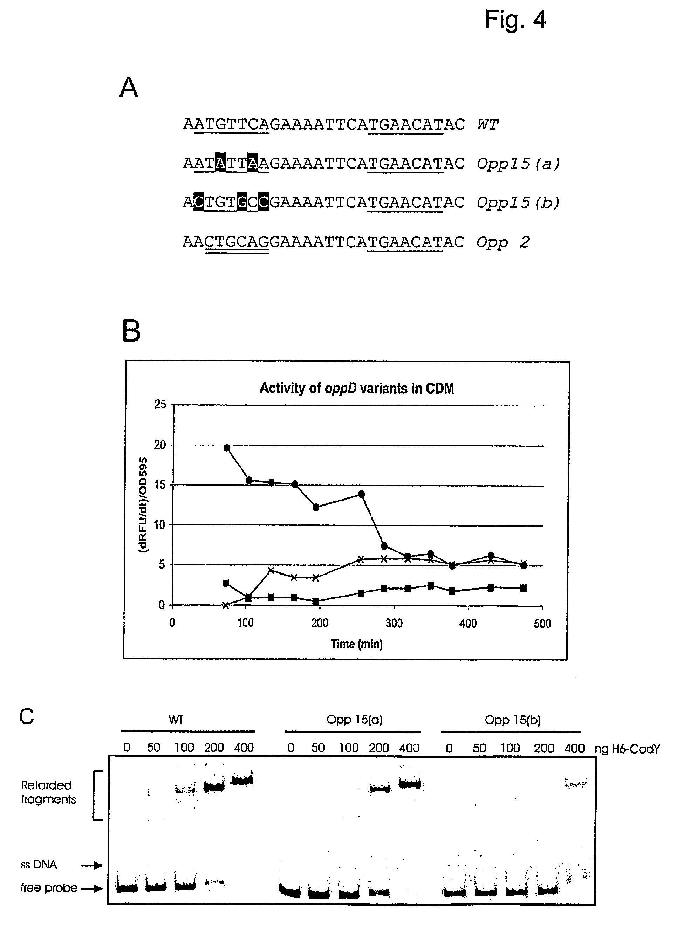

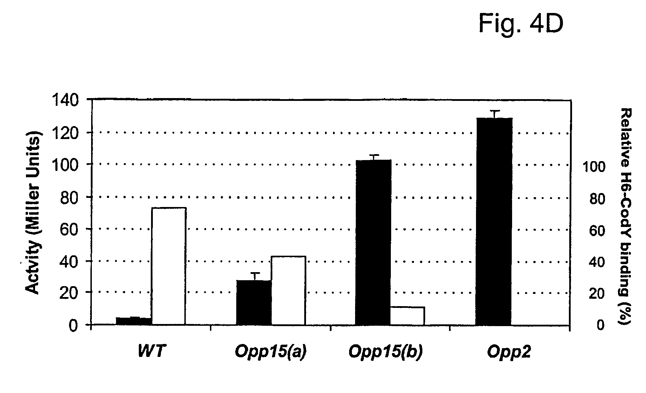

As the fragments used in the EMSA's were also cloned upstream of the promoterless *lacZ* gene in plasmid pORI13, the *in vivo* regulation of a downstream reporter gene (*lacZ*) could be determined by performing β-galactosidase (β-gal) assays in a chemically defined medium (Poolman and Konings, 1988) containing 2% of casitone as a nitrogen source. It was shown that deletion of the sequence abolished medium dependent repression of *lacZ* expression. the fragments were fused upstream of the promoterless *lacZ* gene in plasmid pORI13 (Sanders et al, 1998) and introduced in *L. lactis* LL108. β-Galactosidase activities in exponential phase cells growing in CDM supplemented with 2% casitone, where strong CodY-mediated repression is ensured, correlated with the observed binding pattern (Fig. 3C), indicating that at least part of an operator site for CodY must rely in a region between positions -111 and -68 relative to the *oppD* TSS.
The introduction of base substitutions in the upstream half-site of the inverted repeat gave rise to both weaker binding of H6-CodY and resulted in derepression of the promoter as shown by gel retardation analysis and β-gal assays, respectively (Fig. 4).

### H6-CodY protects an extended region of the oppD promoter.

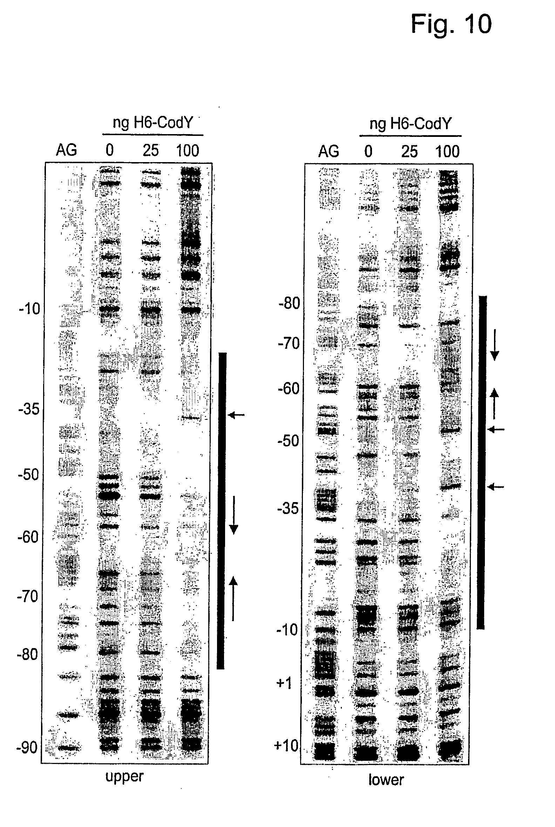

Although the EMSA experiments using truncated *PoppD* DNA fragments pointed to a specific region that is important for CodY binding, the actual area facilitating binding was rather large (position -68 to -111). Moreover, the assays did not exclude the possibility that more downstream sequences could contribute to CodY binding. Therefore, complex formation of H6-CodY with the *oppD* promoter region was investigated by DNaseI footprinting experiments, using the labeled promoter fragment (*opp162*) and binding conditions as used for the EMSAs (Fig. 10). H6-CodY binding results in the protection of bases extending from the -80 to -20 and -80 to -10 positions relative to the transcription start site of the upper and lower strand, respectively. In addition to these protected regions, both DNA strands contained hypersensitive sites when they were incubated with DNaseI in the presence of H6-CodY. These results show that H6-CodY binds to a region encompassing the -35 to -10 sequences of the promoter of *oppD.*

### A region in the oppD promoter containing an inverted repeat is important for CodY-mediated regulation.

A closer inspection of the area in the *oppD* promoter to which CodY binds revealed the presence of a short sequence (ATGTTCA) that is inversely repeated (IR) with a spacing of 9 bp between the partners (Fig. 1). This region of dyad symmetry is located 18 bp upstream of the -35 sequence and is entirely present in probes 2 and 3 to which H6-CodY bound (Fig. 3). The fact that H6-CodY was unable to form a protein-DNA complex when a probe was used that lacks the upstream arm of this IR (probe 1 in Fig. 3) implies that this area might serve as an operator site for CodY on *PoppD.* To study this region in more detail, site directed mutations were introduced by PCR using combinations of oligonucleotide opp1 with oligonucleotides opp14 (WT), opp15(a), opp(15b) or opp2, respectively (Fig. 4A). The PCR products were ligated upstream of the promoterless *lacZ* reporter gene in pORI13 and introduced into *L. lactis* LL108. Introducing base substitutions in the upstream arm of the repeat (*opp15*(*a*) and opp*15*(*b*)) resulted in both weaker binding of H6-CodY (Fig. 4C) and in derepression of P*oppD*-driven *lacZ* expression (Fig. 4D). When the unchanged half-site was replaced by an unrelated sequence (an *Xba*I endonuclease site, present in *opp2*), repression was reduced approximately 30-fold (in cells growing exponentially in rich medium) and H6-CodY binding was completely abolished (as shown in Fig. 3). Converting both the C and G residues in this region to adenines resulted in a reduction of repression of about 7-fold (*opp15*(*a*))*,* whereas changing 3 out of 6 bases (*opp15*(*b*)) led to a derepression of expression of more than 20 times. These results were in accordance with those obtained from gel retardation analyses (Fig. 4C), where the order of affinity of H6-CodY for the probes proved to be: WT probe > *opp15*(*a*) *> opp15*(*b*)*.* In case of the latter probe, hardly any protein-DNA complexes were present and all intermediate complexes that were observed with the *WT and opp15*(*a*) fragments were absent.

### Random mutation analysis of the oppD upstream region.

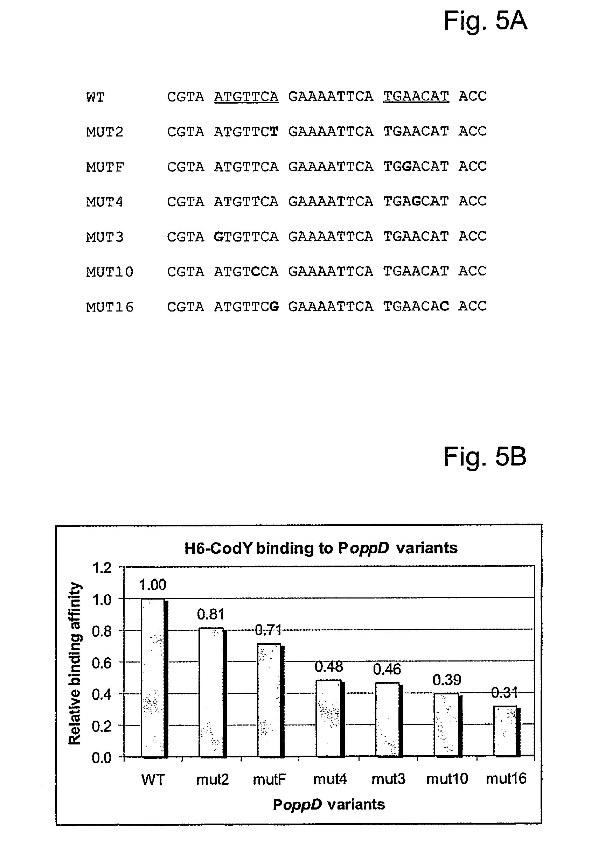

In order to identify bindings sites of CodY in the *oppD* upstream region, random mutation was carried out on the smallest *oppD* promoter fragment that is still bound by CodY (see Fig. 3). By choosing PCR conditions that allow mismatches to occur during DNA amplification, DNA fragments spanning the *oppD* promoter region were obtained containing randomly introduced base pair substitutions. The fragments were restricted using the appropriate restriction enzymes, cloned upstream of the promoterless *lacZ* reporter gene in pORI13 and introduced in *L. lactis* LL108. Transformants that show a derepressed phenotype appear as white or light blue colonies on agarplates containing excess nitrogen sources (2.5% of casitone) and X-gal. Mutants that showed a distorted blue coloring on plates were tested for the ability to complex with CodY in a gel retardation experiment. The strength of binding was determined by comparing the amount of shifted mutated DNA fragment as compared to that of the corresponding wild type fragment (Fig. 5). Weaker binding to all of the mutated promoter fragments was observed. Strikingly, all the mutants obtained carried substitutions in one or both of the half-sites of the inverted repeat present in the *oppD* upstream region, indicating the importance of this sequence in CodY binding. Sequence analysesof the *PoppD* variants obtained in this study revealed that all of them carried one or more base pair substitution(s), of which at least one is located in the region from -82 to -56 relative to the TSS of *oppD.* This, again, is an indication of the importance of this region for CodY binding.

### Identification of additional CodY targets.

In order to identify additional genes that are regulated by CodY, the transcriptional profile of wild-type (WT) *L. lactis* MG1363 was compared with that of a *L. lactis* MG1363 strain containing a 423 bps internal deletion in the *codY gene* using DNA micro arrays containing genes of *L. latis* IL1403. These studies revealed several differentially expressed genes in the delta *codY* strain. The genes of which the expression is increased most significantly in the delta *codY* strain are listed in Table 2. The genes of which the expression is decreased most significantly in the absence of a *CodY*-like protein are listed in Table 3. As this increased expression (Table 2) could be a direct effect of the absence of CodY (derepression), the upstream regions were examined for the presence of conserved nucleotide sequences. It was found that many of these genes contain an upstream sequence that is homologous to the upstream half-site of the palindromic sequence found to be important in *oppD* regulation (Fig. 6A). Interestingly, the upstream region of the gene that shows the highest fold difference in expression when comparing the *codY* strain to WT *L. lactis* MG1363, optS, contains two copies of this sequence. The sequence identified is not in all instances part of an inverted repeat. At a later stage, the experiment was repeated. Results are listed in table 10.

The conserved sequence could not be discerned in the upstream regions of all the differentially expressed genes. This could mean that the altered expression in the delta *codY* strain is an indirect effect of the mutation or that other sequences or structural determinants also play a role in the recognition/regulation by CodY. As can be seen in Fig.7 several other conserved motifs could be identified in the upstream regions of several differentially expressed genes. These sequences could play a role in CodY-regulated expression (e.g. in the case of *prtP* and *prtM).*

Using a weight matrix constructed from the sequences that comprise the putative CodY box, a search was performed in a data set containing the upstream regions of all IL1403 genes (Table 4). The presence of such an element could indicate that the downstream gene is under direct transcriptional control of CodY.

### An overrepresented motif is present in the upstream regions of a number of the CodY-repressed genes

In order to asses whether the lactococcal members of the CodY regulon share a sequence motif in their regulatory regions, an *in silico* sequence analysis was performed using the algorithm of the MEME software tool. Firstly, a dataset was created containing the upstream regions of the genes that were derepressed to the highest extent in the *codY* strain in our DNA microarray experiments, as we assumed that these genes are most likely to be under direct control of CodY and thus contain a CodY binding site. This dataset was supplemented with the upstream regions of genes of which it is known that they are under direct control of CodY (*i*.*e. oppD, pepC, pepN*) as is the case for the intergenic region of the divergently transcribed *L. lactis* subsp. *cremoris* SK11 plasmid-located *prtP* and *prtM* genes for which it has been shown that purified H6-CodY of the closely related lactococcal strain MG1363 is able to interact with DNA fragments spanning this region in *in vitro* DNA binding studies. Since operator sites of regulatory proteins in bacteria are usually located within close proximity of their target promoters, fragments of 200 bps were chosen such that they encompassed the known or predicted promoter sequences of their cognate genes. In cases were a complete operon was found to be up-regulated in the *codY* deletion strain, the upstream region of the first gene of the transcriptional unit was selected (*i.e ilv, his, opp* and *dpp*)*.* Of the latter operon, the region preceding *dppA* was also included in the dataset, since a putative promoter sequence is present in this area that, thus, might contain a CodY binding site as well.
Subsequently, the dataset containing 14 sequences was examined for the occurrence of common elements using the MEME algorithm. As there is no prior knowledge about a possible CodY binding site, MEME was not restricted with respect to the motif width and number of repetitions and allowed to search on either of the given or the reverse DNA strand. Moreover, these settings did prevent that the common upstream elements (-35 and -10 sequences and the ribosome binding site) would conceal the presence of a CodY binding site. As can be seen from Table 4A and Fig. 6B, application of the pattern recognition program approach revealed the presence of a distinct over-represented motif in a number of the DNA sequences of the dataset. Derivates of this 15 bps AATTTTCWGAAAATT inversely repeated (IR) cis-element are present in the upstream regions of 11 out of the 14 co-regulated genes that constitute the input data for the program. In the upstream regions of *udp, hisC,* and *pepN* this over-represented box in CodY-co-regulated genes (C-box) seems to be absent.
Interestingly, the upstream region of the operons that show the highest fold difference in ex-pression when comparing the *codY* strain to WT *L. lactis* MG1363, *dpp* and *ilv,* contain two copies of this C-box preceding both *dppP, dpp* and *ilvD,* encoding the peptide binding proteins of the uptake system and the three branched-chain amino acid biosynthesis genes, respectively, whereas in case of *ctrA* and in front of *gltA,* even 3 copies of the motif can be found.

### Inter-species analysis of the over represented motif

A multi-species string search was performed on several Gram-positive bacteria in order to assess whether the putative CodY box found upstream of the genes belonging to the CodY regulon is also present in other bacteria containing a CodY protein. Such a comparison could reveal subtle differences in the consensus sequence of this motif. As depicted in Fig. 7, the comparison indeed showed that derivatives of the motif are present in the upstream sequences of CodY regulated genes of *B. subtilis* (e.g. *dppA* and *hutP*). Fig. 6A shows a graphical representation of a species-specific "weight matrix" that was built using the aligned sequences of *L. lactis* or *B. subtilis,* respectively. These matrices indicate the importance of specific bases at each position in the motif and show that the motif detected in *L. lactis* seems to be highly similar to the one of *B. subtilis.* Both encompass the same "core" sequence, but the consensus of the *L. lactis* motif seems to be somewhat extended on both sides.

Recently, Molle *et al*. (2003) reported the genome wide expression analysis of a *B. subtilis codY* mutant strain. Using the upstream nucleotide sequences of the targets found in that study, we searched for the presence of the putative CodY box in these sequences. Although the similarity scores with the consensus where not very high, derivates of the motif could be identified in some instances in this set of sequences (Table 5). Analysis of the full genome sequence of *B. subtilis* resulted in the identification of additional promoters containing the putative CodY box (Table 6).

A similar search was performed using the genomes of several other Gram-positive bacteria (Tables 7 and 8). Currently, we are mining the genome of *L. lactis* strain MG1363 of which the genome sequence is nearly completed.

### Regulation of prtP and prtM is derepressed in the CodY-deficient strain.

As *prtP* and *prtM* respond in a similar way to changes in the nitrogen content of the growth medium as CodY-regulated genes (Miladinov, Kuipers, and Topisirovic, 2001;Guedon *et al*., 2001a), the role of CodY *in prtP and prtM* expression was studied. A fragment containing the *prtP*/*prtM intergenic* region of *L. lactis* BGMN1-5 was cloned in between the promoterless *Escherichia coli* β-galactosidase (*lacZ*) and the *Cyamopsis tetragonoloba* α-galactosidase *(α-gal)* genes of pGKH10. Thus, translational fusions of *prtP* and *prtM* with the two reporter genes were created, the AUG codons of *prtP* or *prtM* serving as a start codon for *lacZ* or *α-gal.* In the resulting plasmid pGKB11, *lacZ* is under the control of the *prtP* promoter of BGMN1-5. In this plasmid the *prtM* promoter directs the transcription of the α-gal gene. In the corresponding plasmid pGKB12, the fragment is present in the opposite orientation. Plasmids pGKB11 and pGKB12 were introduced in the CodY-deficient *L. lactis* MG1363 strain (*L. lactis* MG1363*codY*Δ*1*) and *lacZ* expression was analysed in CDM media containing different amounts of casitone, and in peptide-rich M17 medium. In the WT strain, the β-gal activities of the *prtP::lacZ* and *prtM::lacZ* fusions were 6- and 8-fold lower in CDM with 2% than in CDM with 0.2% casitone, respectively. In the *codY* mutant, β-gal activity in CDM with 2% casitone was less than twofold lower than that in CDM with 0.2% casitone. β-gal activities in M17 were similar to those in CDM with 2% casitone for both MG1363 and MG1363*codY*Δ1. These results show that repression of these two gene fusions by medium peptides was almost fully abolished in the *codY* mutant.

### CodY binds to the prtP/prtM intergenic region.

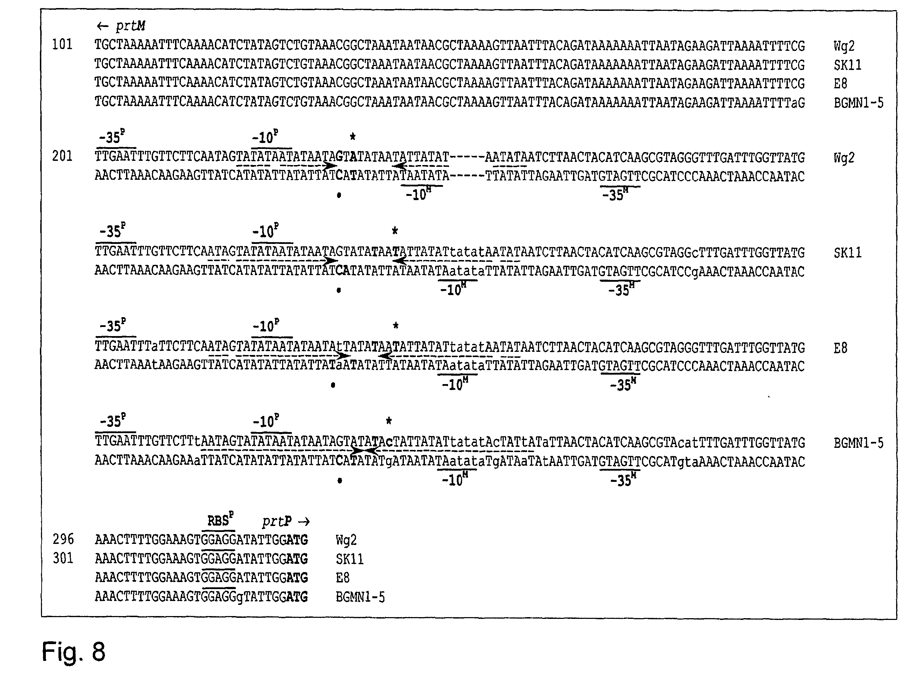

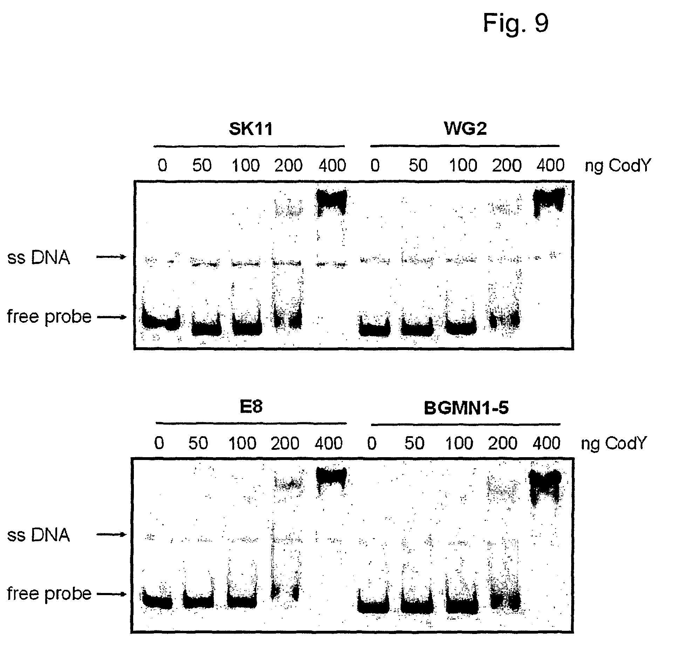

The *prtP*/*prtM* intergenic regions of the *L. lactis* strains BGMN1-5, E8, SK11 and Wg2 each contain an inverted repeat (Fig. 8), that is highly conserved on the sequential level (Marugg *et al*., 1996). To determine whether CodY is also able to recognize and bind to the *prtP*/*prtM* intergenic region, gel mobility shift assays were performed using purified H6-CodY. A 330-bp γ-³²P-labelled PCR fragment containing the *prtPlprtM* intergenic region derived from *L. lactis* BGMN1-5, E8, SK11 and Wg2, respectively, were used as probes. Addition of His6-CodY resulted in a markedly lower electrophoretic mobility of all four double stranded PCR products on a polyacrylamide gel as compared to the situation in which CodY was not added (Fig. 9). Moreover, multiple shifted bands are present suggesting that CodY migh act as a multimer. These results indicate that CodY directly binds to the *prtP*/*prtM* intergenic regions of all four lactococcal strains tested. Since the inverted repeats present in the *prtPlprtM* intergenic region overlaps the -10 sequence of both the *prtP and prtM* promoter, they could function as a binding site for CodY, thereby blocking transcription of both genes simultaneously.

### H6-CodY forms multimers in the absence of GTP.

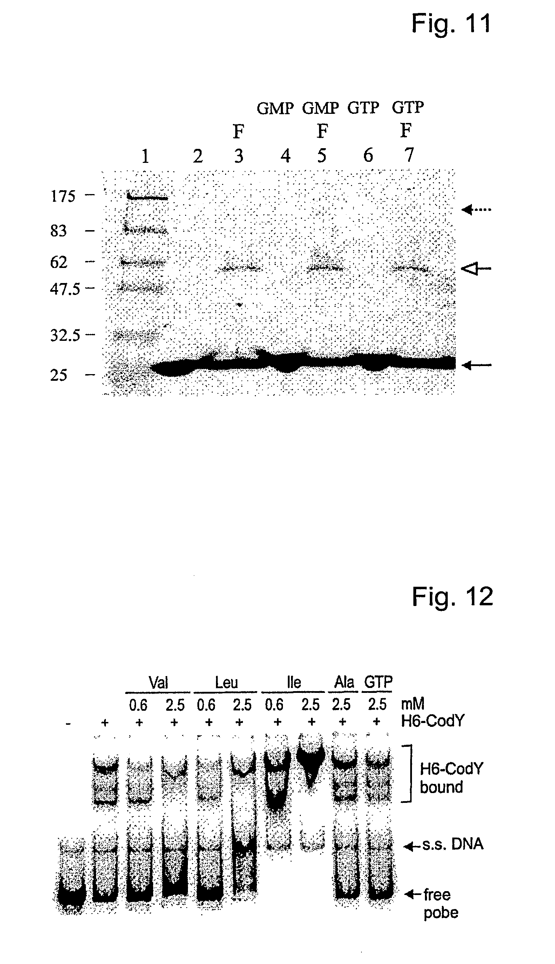

In order to identify whether the active form of H6-CodY, a protein of 29 kDa, consists of mono- or multimers, cross-linking experiments were carried out on purified H6-CodY. Initial experiments, using glutaraldehyde as a cross-linking reagent proved to be unsuccessful (data not shown). The results using formaldehyde as a cross-linking agent are shown in figure 11 and show that oligomeric forms of H6-CodY are present. Covalently linked CodY complexes of sizes close to those expected for dimeric (58 kDa) and, less pronounced, tetrameric H6-CodY molecules (116 kDa) were obtained. Formation of cross-linked H6-CodY complexes was not significantly stimulated by the presence of GTP, a molecule that serves as a cofactor of *B. subtilis* CodY. These results indicate that the native form of CodY is a di- or tetramer.

### BCAAs affect H6-CodY binding properties. 22

Evidence has been presented that CodY senses the nitrogen supply of the cell as a function of the BCAA pool. Although BCAAs might act as direct effectors of CodY activity, the exact nature of this signal remains to be established. Therefore, *in vitro* DNA binding of H6-CodY was examined in the presence or absence of the three BCAAs Val, Leu and Ile (figure 12). The addition of any of the three BCAAs resulted in severely altered binding of H6-CodY to the *PoppD* probe 3 as compared to the situation in which no or another, aliphatic, amino acid (*i.e*. alanine) was present. All of the probe DNA was retarded in the presence of Ile at concentrations of both 0.6 and 2.5 mM, showing that this amino acid directly stimulates the binding of H6-CodY to its target. Strikingly, in the presence of Val and Leu, H6-CodY behaved rather differently. The addition of either of these BCAAs to a final concentration of 0.6 mM resulted in a reduction of the amount of shifted, H6-CodY-bound, probe DNA. Increasing the concentration of these two amino acids to 2.5 mM led to the formation of a protein-DNA complex of high molecular weight, whereas the capability of H6-CodY to form complexes of lower molecular weight seems to be lost.
Similarly, we also tested whether GTP could stimulate the binding of H6-CodY to lactococcal *PoppD,* since in *B. subtilis* GTP serves as an effector molecule that enhances binding of bacillus CodY to a number of its targets. However, no effect is seen on lactococcal CodY at a concentration of 2.5 mM GTP (right lane in figure 12) as well as at other concentrations (data not shown).

### CodY directly complexes with the asnB and ctrA upstream regions

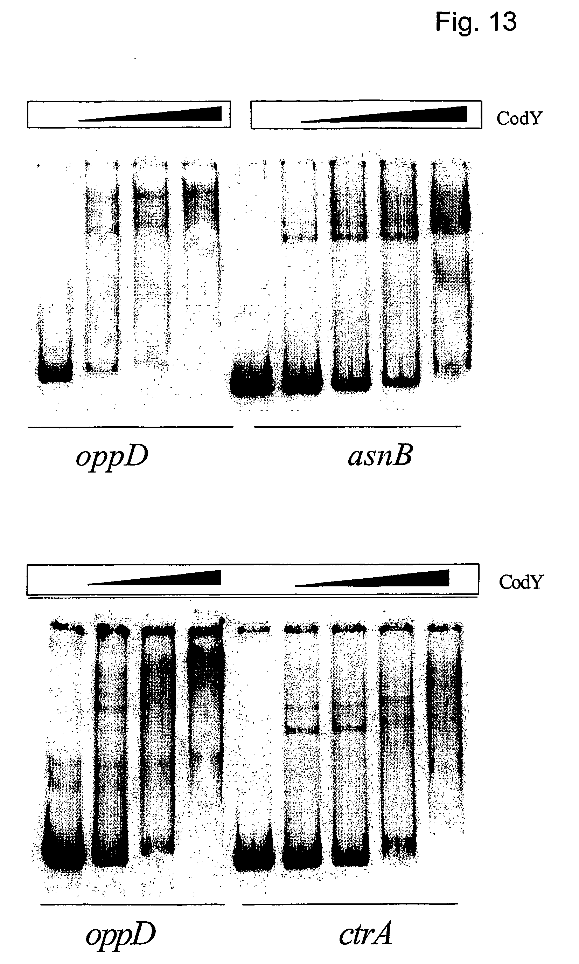

To distinguish whether repression by CodY occurs directly or indirectly, an electric mobility shift assay (EMSA) was performed in which purified H6-CodY was used. Figure 13 shows that H6-CodY, like in the case of the well-studied *oppD* promoter, interacted with a radioactively labeled DNA probe spanning the *asnB promoter in vitro.*
This result, together with those of the *in vivo* transcription assays indicate that expression of *asnB* is probably under the direct control of CodY. Using EMSA's, direct interaction of H6-CodY was also shown for a probe encompassing the upstream region of *ctrA.* As is apparent from Table 2 and Table 10, the transcript level of *ctrA,* a gene encoding a putative transporter with homology to several cationic amino acid permeases is highly elevated in *L. lactis* MG1363Dcody).

### DESCIPTION OF FIGURES

**Figure 1** Overview of the *opp-pepO1* operon.
   Panel A shows a schematic overview of the *opp pepO1 operon.* Panel B shows a detailed view of the upstream sequence encompassing the *oppD* promoter. The -35, -10 and ribosome binding site (RBS) sequences are underlined. The arrows show the positions of the two pairs of inverted repeats. The inverted repeat that is discussed in the text in more detail is indicated in bold.
**Figure 2** His6-CodY binds to the *oppD* promoter.
   Gel retardation assay using His6-CodY and the *oppD* promoter fragment. A DNA fragment encompassing the *oppD* promoter was amplified by PCR, radioactively labelled and incubated with no (lane 2) or increasing amounts of purified H6-CodY (lanes 3 to 9). Lane 1 contains the same probe, but was boiled in a 95% formamide solution in order to obtain single stranded DNA fragments.
**Figure 3** Determination of the minimal region involved in CodY binding to the *oppD* upstream promoter region.
   Different parts of the *oppD* promoter region were amplified by PCR (panel A), radioactively labelled and incubated with no (lanes 1), 20 ng (lanes 2) or 200 ng (lanes 3) of His6-CodY protein, respectively (panel B).
   Figure 3C: *In vivo* activity of P*oppD* variants. *L. lactis* LL108 strains carrying *lacZ* reporter plasmids fused the opp fragments depicted in panel A were grown in CDM containing 2% casitone. Cells were harvested in the exponential phase of growth and ß-galactosidase activity was measured (solid bars). The experiments were carried out in triplicate. Error bars indicate standard deviations.
**Figure 4** Effects of site-directed mutations in the area of inverted repeat in the *oppD* upstream region. Three constructs (panel A) were compared for both β-gal activity (panel B) and H6-CodY binding (panel C). WT fragments contain no substitutions, whereas fragments Opp15 (a) and Opp15 (b) contain 2 and 3 mutations, respectively (indicated in bold in panel A).In opp2 an XbaI endonuclease site (doubly underlined) preceded by two adenine residues replaced the complete left arm. Solid lines mark the inverted repeats. Panel B shows the effects of mutations on *in vivo lacZ* expression during growth using WT (■), Opp15 (a) (x) and Opp15 (b) (•) *lacZ* fusion constructs. Activity is depicted as the change of fluorescence per unit time as function of the optical density.
   Figure 4D: Promoter activity of P*oppD* variants. *L. lactis* LL108 strains carrying the *lacz* reporter plasmids were grown in GM17 medium. Cells were harvested in the exponential phase of growth and β-galactosidase activity was measured (solid bars). The experiments were carried out in triplicate. Error bars indicate standard deviations. Open bars indicate a quantitative representation of the DNA binding assay shown in panel C. The relative binding affinity of H6-CodY for the P*oppD* variants was calculated by comparing the intensity of the shifted, H6-CodY bound complexes with the total radioactive signal in each lane in the presence of 100 ng protein.
**Figure 5** H6-CodY binding to fragments of derepressed variants of *lacZ* fusion constructs.
   Panel A shows the positions of the basepair substitutions (indicated in bold) of the mutants that showed distorted repression by CodY relative to the WT. Solid lines mark the inverted repeat. Panel B shows the relative binding of H6-CodY (compared to WT) to labelled PCR products encompassing the promoter region of the mutants in an in *vitro* binding assay. The relative affinity was calculated by comparing the amount of H6-CodY required to shift 50% of the labelled DNA in the binding assay.
**Figure 6A** Weight matrices as deduced from the seperate motifs as observed in *L. lactis* intergenic (upper part) and *B. subtilis* (lower part). IUPAC codes of the derived consensus are indicated in bold.
   **Figure 6B** Weight matrix obtained by pattern recognition program approach.
**Figure 7** Several conserved motifs that can be discerned in the upstream regions of CodY-regulated genes. Motif 10 represents the consensus CodY target sequence as depicted in Figure 6.
**Figure** 8 Comparison of the *prtP*/*prtM promoter* regions of *L. lactis* strains Wg2, SK11, E8 and BGMN1-5. The regions between bp 201 and 296 are shown double stranded for each strain, the other (flanking) regions are single stranded. Differences in the nucleotide sequences between the strains are depicted in lower case. *prtP* and *prtM* start codons are indicated in bold and with a small arrow. The deletion in the sequence of *L. lactis* Wg2 is indicated by a dashed line. The major transcription start sites for *prtP* (*) *and prtM* (•) are indicated above and below the sequence, respectively. Minor sites are indicated in bold face. Putative ribosomal binding sites (RBS^{P}, RBS^{M}) and -10 (-10^{P}, -10^{M}) and -35 (-35^{P}, -35^{M}) promoter sequences are overlined. Dashed arrows represent inverted repeats overlapping the promoters.
**Figure 9** Binding of H6-CodY to the *prtP*/*prtM* intergenic regions of *L. lactis* strains BGMN1-5, E8, SK11 and Wg2. The respective labelled DNA fragments were incubated with increasing amounts of CodY protein (as indicated above the gels) and subjected to gel electrophoresis. The positions of single stranded DNA (ss DNA) and free probe are indicated in the left margin.
**Figure 10** DNaseI footprinting analysis of H6-CodY binding to the *oppD* promoter region. The left and right panels show the footprint of the upper and lower strand, respectively. Footprints using radioactively labeled probe 3, obtained in the absence or presence of 25 or 100 ng of H6-CodY, are flanked by a Maxam and Gilbert A+G sequence ladder (AG) on the left. Numbers on the left indicate basepair positions relative to the transcriptional start site. Protected regions are marked with bars and horizontal arrows indicate the positions of hypersensitive bonds. The vertical arrows indicate the region of dyad symmetry closest to the -35 sequence (see Fig. 1B).
**Figure 11** Detection of cross-linked H6-CodY multimers. Samples containing H6-CodY were incubated at 30°C in the presence (marked by an F) or absence of 1% formaldehyde. GMP was added to the samples in lanes 4 and 5; GTP was added to samples in lanes 6 and 7. Lane 1 contains a molecular weight marker of which the sizes, in kilodaltons, are indicated in the left margin. The positions of monomeric, dimeric and tetrameric forms of H6-CodY are indicated in the right margin by closed, open and dotted arrows, respectively.
**Figure 12** EMSA analysis of the effects of BCAAs and GTP on H6-CodY binding to P*oppD*. Radioactively labeled probe 3 (see Fig. 3A) was incubated alone or in the presence of 50 ng of purified H6-CodY (marked by - or + signs, respectively). Where indicated, samples were incubated in the presence of 0.6 or 2.5 mM of Val, Leu, Ile and GTP, respectively. The positions of free probe, single stranded DNA and CodY-probe complexes are indicated in the right margin.
**Figure 13** Binding of H6-CodY to radioactively labeled DNA probes spanning the *oppD, asnB* promoter and *ctrA* upstream region.

### TABLES

**Table 1. Comparison of putative GTP binding motifs in CodY homologs**

| Small GTPases | G1 | G3 | G4 |
|---|---|---|---|
| Consensus sequences | GXXXXGXT A S | DXXG TQ | NKXD |
| FtsZ | | | |
| *E. coli* | LGGGTGTG | DAFG | TSLD |
| CodY | | | |
| *B. subtilis* | GGERLGTL | DRVG | NKFL |
| *B. stearothermophilus* | GGERLGTL | DRVG | DKFL |
| *B. halodurans* | GGQRLGTL | DRVG | DKFL |
| *B. anthracis* | GGERLGTL | NA^{a} | NA^{a} |
| *C. difficile* | GGMRLGSL | DRIG | NEGI |
| *C. acetobutylicum* | NRERLGTL | DRVG | ILND |
| *S. pneumoniae* | SGIRLGSL | DRIG | LISD |
| *E. faecalis* | AGKRLGTI | DRVG | NQQF |
| *S. mutans* | GGMRLGSL | DRIG | NEGI |
| *S. aureus* | GGERLGTL | DRIG | EKGI |
| *S. pyogenes* | GGMRLGSL | DRIG | NEGI |
| *L. lactis* | SGMRLGTF | DKIG | TGLF |

| | | | |
|---|---|---|---|
| ^{a}(uit Ratnayake-Lecamwasam *et al*., 2001), NA; not available. | | | |

**Table 2. Genes up in CodY strain**

| **Genes up in** | | |
|---|---|---|
| **dcodY** | **Fold** | **Annotation** |
| optS | 8.8 | oligopeptide ABC transporter substrate binding protein |
| ctrA | 6.3 | cationic amino acid transporter |
| pepO | 5.7 | neutral endopeptidase |
| citB | 5.6 | aconitate hydratase (EC 4.2.1.3) |
| oppA | 5.3 | oligopeptide ABC transporter substrate binding protein |
| gltD | 5.2 | glutamate synthase small subunit (EC 1.4.1.13) |
| ilvD | 4.8 | dihydroxy-acid dehydratase (EC 4.2.1.9) |
| ilvN | 4.5 | acetolactate synthase small subunit (EC 4.1.3.18) |
| asnB | 4.4 | asparagine synthetase B |
| ymdC | 3.9 | kanamycin kinase (EC 2.7.1.95) |
| hisA | 3.8 | phosphoribosylformimino-5-aminoimidazole isomerase |
| oppB | 3.4 | oligopeptide ABC transporter permease protein |
| oppC | 2.9 | oligopeptide ABC transporter permease protein |
| oppF | 2.8 | oligopeptide ABC transporter ATP binding protein |
| llrH | 2.7 | two-component system regulator |
| hisB | 2.7 | imidazoleglycerol-phosphate dehydratase (EC 4.2.1.19) |
| serC | 2.6 | phosphoserine aminotransferase (EC 2.6.1.52) |
| hisK | 2.5 | histidinol phosphatase |
| oppD | 2.5 | oligopeptide ABC transporter ATP binding protein |
| yahD | 2.4 | HYPOTHETICAL PROTEIN |
| leuC | 2.3 | 3-isopropylmalate dehydratase large subunit (EC 4.2.1.33) |
| optD | 2.3 | oligopeptide ABC transporter ATP binding protein |
| serB | 2.2 | phosphoserine phosphatase (EC 3.1.3.3) |
| hisH | 2.1 | amidotransferase (EC 2.4.2.-) |
| udp | 2.1 | uridine phosphorylase |
| aldB | 2.0 | alpha-acetolactate decarboxylase (EC 4.1.1.5) |
| hisI | 2.0 | phosphoribosyl-ATP pyrophosphohydrolase (EC 3.6.1.31) |
| icd | 1.9 | isocitrate dehydrogenase (EC 1.1.1.42) |
| yafC | 1.9 | HYPOTHETICAL PROTEIN |
| arcD1 | 1.8 | arginine/ornitine antiporter |
| ilvB | 1.8 | acetolactate synthase large subunit (EC 4.1.3.18) |
| optF | 1.8 | oligopeptide ABC transporter ATP binding protein |
| lacR | 1.8 | lactose transport regulator |
| hisD | 1.7 | histidinol dehydrogenase (EC 1.1.1.23) |
| arcC2 | 1.7 | carbamate kinase (EC 2.7.2.2) |
| optA | 1.7 | oligopeptide ABC transporter substrate binding protein |
| serA | 1.7 | D-3-phosphoglycerate dehydrogenase (EC 1.1.1.95) |
| recD | 1.7 | exodeoxyribonuclease V alpha chain (EC 3.1.11.5) |
| ilvC | 1.7 | ketol-acid reductoisomerase (EC 1.1.1.86) |
| ywaD | 1.7 | UNKNOWN PROTEIN |
| ydbD | 1.7 | UNKNOWN PROTEIN |
| glgD | 1.7 | glucose-1-phosphate adenylyltransferase (EC 2.7.7.27) |
| lmrP | 1.6 | integral membrane protein LmrP |
| pdhC | 1.6 | component of PDH complex (EC 2.3.1.12) |
| mesJ | 1.6 | cell cycle protein MesJ |
| lcnD | 1.6 | lactococcin A ABC transporter permease protein |
| glgA | 1.6 | glycogen synthase (EC 2.4.1.21) |
| trxA | 1.6 | thioredoxin |
| gltA | 1.6 | citrate synthase (EC 4.1.3.7) |
| pepN | 1.6 | aminopeptidase N |
| areA | 1.6 | arginine deiminase (EC 3.5.3.6) |
| glgC | 1.6 | glucose-1-phosphate adenylyltransferase (EC 2.7.7.27) |
| yohC | 1.6 | transcriptional regulator |
| ywiE | 1.6 | UNKNOWN PROTEIN |
| yohD | 1.6 | UNKNOWN PROTEIN |
| yfiD | 1.5 | UNKNOWN PROTEIN |
| ycaF | 1.5 | UNKNOWN PROTEIN |
| ybhE | 1.5 | HYPOTHETICAL PROTEIN |
| rmlA | 1.5 | glucose-1-phosphate thymidylyltransferase (EC 2.7.7.24) |
| grpE | 1.5 | stress responce protein GrpE |
| yccE | 1.5 | UNKNOWN PROTEIN |
| hrcA | 1.5 | heat-inducible transcription repressor HrcA |
| pi336 | 1.5 | prophage pi3 protein 36 |
| dhaM | 1.5 | dihydroxyacetone kinase (EC 2.7.1.2) |
| ywjC | 1.5 | UNKNOWN PROTEIN |
| purC | 1.5 | phosphoribosylaminoimidazole synthetase |
| xynB | 1.5 | beta-1,4-xylosidase (EC 3.2.1.37) |
| lysA | 1.5 | diaminopimelate decarboxylase (EC 4.1.1.20) |
| yccF | 1.5 | HYPOTHETICAL PROTEIN |
| rmaB | 1.4 | transcriptional regulator |
| optC | 1.4 | oligopeptide ABC transporter permease protein |
| araT | 1.4 | aromatic amino acid specific aminotransferase |
| yciA | 1.4 | amino acid amidohydrolase |
| pi235 | 1.4 | prophage pi2 protein 35 |
| yfgC | 1.4 | UNKNOWN PROTEIN |
| pepC | 1.4 | aminopeptidase C |
| yaiB | 1.4 | HYPOTHETICAL PROTEIN |
| ybhD | 1.4 | UNKNOWN PROTEIN |
| ribA | 1.4 | GTP cyclohydrolase II (EC 3.5.4.25) |
| pyrG | 1.4 | CTP synthetase |
| ps120 | 1.4 | prophage ps1 protein 20 |
| yidA | 1.4 | transcription regulator |
| yahC | 1.4 | UNKNOWN PROTEIN |
| ps305 | 1.4 | prophage ps3 protein 05 |
| yndG | 1.4 | metal ABC transporter substrate binding protein |
| yahG | 1.4 | ABC transporter ATP binding protein |
| uxuA | 1.4 | D-mannonate dehydratase (EC 4.2.1.8) |
| ps112 | 1.4 | prophage ps1 protein 12 |
| hisC | 1.4 | histidinol-phosphate aminotransferase (EC 2.6.1.9) |
| arcC1 | 1.4 | carbamate kinase (EC 2.7.2.2) |
| xylX | 1.4 | acetyltransferase HYPOTHETICAL PROTEIN |
| rodA | 1.4 | rod-shape determining protein |
| yafB | 1.4 | sulfate transporter |
| ps113 | 1.4 | prophage ps1 protein 13 |
| ruvA | 1.4 | DNA helicase RuvA |
| asd | 1.4 | aspartate-semialdehyde dehydrogenase (EC 1.2.1.11) |
| sugE | 1.4 | SugE protein |
| groES | 1.4 | 10 KD chaperonin |
| rpsO | 1.4 | 30S ribosomal protein S15 |
| ybdA | 1.4 | transcription regulator |
| bcaT | 1.4 | branched-chain amino acid aminotransferase (EC 2.6.1.42) |
| ybiK | 1.4 | UNKNOWN PROTEIN |
| yafJ | 1.4 | HYPOTHETICAL PROTEIN |
| pi302 | 1.4 | prophage pi3 protein 02 |
| aldR | 1.4 | regulatory protein AldR |
| menX | 1.4 | protein in menaquinone biosynthesis pathway |
| dapB | 1.4 | dihydrodipicolinate reductase (EC 1.3.1.26) |
| yphI | 1.4 | UNKNOWN PROTEIN |
| ydcG | 1.3 | transcriptional regulator |
| amtB | 1.3 | ammonium transporter |

**Table 3. Genes down in CodY strain**

| **Genes down in** | | |
|---|---|---|
| **dcodY** | **Fold** | **Annotation** |
| plpA | 3.3 | outer membrane lipoprotein precursor |
| cysK | 3.2 | cysteine synthase (EC 4.2.99.8) |
| metB2 | 2.9 | cystathionine gamma-synthase (EC 4.2.99.9) |
| plpB | 2.9 | outer membrane lipoprotein precursor |
| cysD | 2.6 | O-acetylhomoserine sulfhydrylase |
| plpC | 2.5 | outer membrane lipoprotein precursor |
| plpD | 2.3 | outer membrane lipoprotein precursor |
| yndE | 2.3 | UNKNOWN PROTEIN |
| cysM | 2.1 | cysteine synthase (EC 4.2.99.8) |
| yrbB | 2.0 | HYPOTHETICAL PROTEIN |
| yjgC | 2.0 | amino acid ABC transporter substrate binding protein |
| argH | 1.8 | argininosuccinate lyase (EC 4.3.2.1) |
| lysP | 1.8 | lysine specific permease |
| gntK | 1.8 | gluconate kinase (EC 2.7.1.12) |
| codY | 1.7 | transcriptional regulator |
| yshA | 1.7 | amino acid permease |
| ymgI | 1.6 | UNKNOWN PROTEIN |
| ytaA | 1.6 | conserved hypothetical protein |
| panE | 1.6 | ketopantoate reductase (EC 1.1.1.169) |
| yhcE | 1.6 | conserved hypothetical protein |
| ywdD | 1.5 | UNKNOWN PROTEIN |
| ymbC | 1.5 | UNKNOWN PROTEIN |
| rpmD | 1.5 | 50S ribosomal protein L30 |
| ywjA | 1.5 | UNKNOWN PROTEIN |
| ynfG | 1.5 | HYPOTHETICAL PROTEIN |
| ybiD | 1.5 | HYPOTHETICAL PROTEIN |
| ydcB | 1.5 | amino acid ABC transporter ATP binding protein |
| ypjC | 1.5 | UNKNOWN PROTEIN |
| ytjE | 1.5 | aminotransferase |
| pydA | 1.5 | dihydroorotate dehydrogenase A (EC 1.3.3.1) |
| ymgG | 1.5 | HYPOTHETICAL PROTEIN |
| nrdG | 1.4 | ribonucleoside-triphosphate reductase activating protein |
| yxeA | 1.4 | HYPOTHETICAL PROTEIN |
| yueD | 1.4 | conserved hypothetical protein |
| ymbK | 1.4 | UNKNOWN PROTEIN |
| ylfH | 1.4 | N-acetylglucosamine catabolic protein |
| yqcA | 1.4 | UNKNOWN PROTEIN |
| yudI | 1.4 | HYPOTHETICAL PROTEIN |
| yvdF | 1.4 | amino acid ABC transporter substrate binding protein |
| yxaF | 1.4 | HYPOTHETICAL PROTEIN |
| ymbJ | 1.4 | UNKNOWN PROTEIN |
| ynfH | 1.4 | UNKNOWN PROTEIN |
| yxbE | 1.4 | conserved hypothetical protein |
| dxsA | 1.4 | 1-deoxyxylulose-5-phosphate synthase |
| murI | 1.4 | gracemase (EC 5.1.1.3) |
| ytbC | 1.4 | UNKNOWN PROTEIN |
| yccB | 1.4 | UNKNOWN PROTEIN |
| yhcH | 1.4 | HYPOTHETICAL PROTEIN |

**Table 4. Whole genome search for the presence of the putative CodY box in intergenic regions of L. lactis IL1403.**

| **Sequence** | **Gene** | **Despription** |
|---|---|---|
| TAATTTTCTGATAATATAGTCAATTT | yreE | UNKNOWN PROTEIN |
| TAATTTACTGACAAGTCTGTCAGTAA | ctrA | cationic amino acid transporter |
| TAATTTACTGACAAAATTATCAGAAC | yciC | HYPOTETICAL PROTEIN |
| AAATTTTCTGACAATAATAAAAATTG | optA | oligopeptide ABC transporter substrate binding protein |
| AAATTATCAGAAAAATACAACAATAT | optS | oligopeptide ABC transporter substrate binding protein |
| TAATTTTCAGAATAATATGAAAATTC | optS | oligopeptide ABC transporter substrate binding protein |
| TAATTTACTGATAGATTTGTCAGTAA | parA | chromosome partitioning protein |
| TAATTTACTGACAGTTCTGTCAGTAA | aroF | Tyr-sensitive aldolase |
| AAATTTACTGACAAAAAAGATAATGG | vacB1 | RIBONUCLEASE II (RNB) FAMILY |
| TAATTTTCAGAAAACATAACCATTAT | optA | oligopeptide ABC transporter substrate binding protein |
| GAATTTTATGAAAAAAATATTAATTG | ypaG | HYPOTHETICAL PROTEIN |
| GAATTTACTGACGAATCTATCATTAA | yiaB | oxidoreductase |
| TCATTCTCTGACAAATCTGTCAGTAA | ysdC | HYPOTHETICAL PROTEIN |
| AAATTTACTGACAAGCTTGTTAGTAT | hemK | protoporphyrinogen oxidase |
| AAATTTAATGATAAAACAATTAGTTT | prfC | peptide chain release factor 3 |
| AAAGTTACTGACAAATCTGTCAGTAA | yugB | ORF |
| TTATTTACTGACAAGTCTGTCAGTAA | murD | PEPTIDOGLYCAN BIOSYNTHESIS |
| TATTTTACTGACAAAAAAATAAGTTT | ywdG | HYPOTHETICAL PROTEIN |
| TAATTTACTGACAGCTTTGTCAGTAA | parC | topoisomerase IV subunit B (EC 5.99.1.-) |
| AAATTTACTGACAGAGCTGTCAGTAA | pepC | aminopeptidase |
| AAATTTACTGACAGACTTGTTAGTAA | mutM | formamidopyrimidine-DNA glycosylase (EC 3.2.2.23) |
| AAATTTACTGACAACTTTGTCAGAAG | rgpAB | rhamnosyltransferase |
| AAAATGTCTGATAAAATGATTAATAC | vacB1 | RIBONUCLEASE II (RNB) FAMILY |
| TAATTTACTGACAGAATTTTAAATTT | recN | DNA repair protein |
| AAAATTACTAACAAAACTGTTAGTAA | llrH | two-component system regulator |

| | | |
|---|---|---|
| Detected motifs are sorted according to their similarity to the consensus | | |

**Table 4A Search of motif in L. lactis MG1363 genome**

| **Position** | **Sequence** | **Score** | **Gene** |
|---|---|---|---|
| 352819 | AATTTTCAGAAAATT | 11.8 | dppP |
| 164084 | AATTGTCAGAAAATT | 11.3 | codY |
| 558564 | AATTTTCTGATAATT | 11.3 | serC |
| 1799629 | AATTTTCAGATAATT | 11.3 | thrA |
| 1860656 | ATTTTTCAGAAAATT | 11.3 | ygjD |
| 2150244 | AATTTTCGGAAAAAT | 11.3 | ytjF |
| 628910 | AATTTTCAGAAAATA | 11.2 | gltA |
| 191904 | ACTTTTCAGAAAATT | 11.1 | feoA |
| 636386 | AATTTTCTGAATATT | 11.1 | yghD |
| 1516191 | ATTTTTCAGAAAAAT | 11.1 | nrdF |
| 112095 | AATTGTCAGACAATT | 10.9 | ctrA |
| 351027 | ATTTTTCTGACAATT | 10.9 | dppA |
| 683034 | AATTTTCTGAACATT | 10.9 | oppD |
| 2150244 | ATTTTTCCGAAAATT | 10.9 | ytjF |
| 279800 | TATTTTCAAAAAATT | 10.8 | ychG |
| 351095 | ATTATTCTGAAAATT | 10.8 | dppA |
| 383134 | TATTTTCAAAAAATT | 10.8 | lyss |
| 1474895 | AATTTTTTGAAAATT | 10.8 | ORF |
| 1815549 | AAATTTCTGAAAATT | 10.8 | rliC |
| 353051 | AATTTTCTGACAATA | 10.7 | dppP |
| 416868 | ATTTTTCTGAAAATA | 10.7 | yeaG |
| 742665 | ATTTTTCAGAAAATA | 10.7 | pip |
| 884469 | GTTTTTCTGAAAATT | 10.7 | yqcE |
| 1809077 | AATTTTCAGAAGAAT | 10.7 | menD |
| 16128 | AATTTTCTAAAAAGT | 10.6 | mfd |
| 352983 | AATTTTCAGAAAACA | 10.6 | dppP |
| 547027 | TATTTTCAAAAAAT | 10.6 | yfhC |
| 868871 | CATCTTCAGAAAATT | 10.6 | arsC |
| 876166 | AATATTCAGAAAATA | 10.6 | amtB |
| 939654 | AATTTTCAAAAAACT | 10.6 | purL |
| 1001446 | AATTTTCAGAAAAGA | 10.6 | yojB |
| 1251475 | AATGTTCTGACAAAT | 10.6 | ilvD |
| 1920664 | AATTTTTTGAAAAAT | 10.6 | ysbD |
| 1940975 | AATTTTTTGAAAAAT | 10.6 | ysdE |
| 2105722 | AATTTTCTAAA.AACT | 10.6 | ORF |
| 100225 | ATTTTACAGAAAATT | 10.5 | ybaB |
| 126604 | AATTATCAGAAAATG | 10.5 | ORF |
| 419279 | AATTATCAGAAAATC | 10.5 | yebA |
| 479710 | ATTTTTCTGAAAATG | 10.5 | ORF |
| 501762 | AATTTTCTGACGATT | 10.5 | cysM |

**Table 5. Presence of putative CodY box upstream of CodY regulated genes identified by Molle et al. 2003.**

| **gene** | **sequence** |
|---|---|
| yufN | ATTATCAGAAAATTT |
| citB | ATTGTGAGAAAATTG |
| dppA | TTTGTTAGAATATTC |
| hutP | GTTATCAGAATTTTT |
| yxbC | ATTATCAGAGGATTA |
| yurPQ | AATTTCAGAAAATAA |
| YcgM | ATTTTGAGGATATTG |
| yhjC | AATTTCAGACAATTC |
| ybgE | ATATTCTGAAATTTA |
| ykbA | TTTATCAAAAAAGTC |
| ggaA | ATTTTCAGCAAAAAA |
| yCgM | ATAATCAGAATCTTT |
| yoaD | TTTTTATGAAAAATA |
| guaB | GTTATCTAAATATTT |
| ilvD | ATTGTCAAAATAAAA |
| yxbBC | ATTGACAGAATTATC |
| roCA | TTTTTCAGCAAAAGA |
| yhdG | TTTTTCTAACAATTT |
| yusC | TTTTGCAGAAAAAAC |
| yusC | TTTCTTAGAATAGTG |

| | |
|---|---|
| Detected motifs are sorted according to their similarity to the consensus | |

**Table 6. Whole genome search for the presence of the putative CodY box in intergenic regions of B. subtilis 168.**

| **Sequence motif** | **Gene** | **Annotation** |
|---|---|---|
| ATTATCAGAAAATTT | yufN | similar to ABC transporter (lipoprotein) |
| ATTTTCAGAAATTTA | ydjJ | function="unknown & similar to sugar transporter |
| TTTTTCAGAAAAATG | ytkC | similar to autolytic amidase |
| TTTTTCAGAAAAATC | lytE | alkaline phosphatase A & cell wall lytic activity |
| ATTGTCTGAATATTA | yoaC | similar to phosphoglycerate dehydrogenase |
| TTTTTCTGAATATTC | ytnA | similar to proline permease |
| ATTTTCGGAAAATTT | aldY | aldehyde dehydrogenase |
| ATTTTCAGAAAAGTT | glnQ | glutamine ABC transporter |
| ATTTTCAGAAAATAA | ykuW | function="unknown |
| ATTTTCAGTATATTT | yndN | |
| TTTATCAGAAAAATA | yheI | |
| ATTTTCAGGAAATTC | ykuM | similar to transcriptional regulator (LysR family)" |
| ATTTTCAGAACAATT | gbsA | similar to hypothetical proteins |
| ATTTTCAGAAAATCA | ynaC | function="unknown |
| ATTGTCAGAAAACTT | yqiQ | similar to phosphoenolpyruvate mutase |
| ATTTTCAGAATTATA | ggaA | membrane-bound protein |
| TTTTTCGGAATATTC | yurY | similar to ABC transporter (ATP-binding protein)" |
| TTTATCTGAAAATTT | ureA | urease (beta subunit)" |
| ATATTCAGAATATTC | leuS | leucyl-tRNA synthetase |
| ATTTTCTGAAATTTC | yurO | similar to multiple sugar-binding protein |
| ATTTTCTGAAATTTA | ykrQ | similar to hypothetical proteins |
| TTTTTCAGTATATTT | yvcC | similar to ABC transporter (ATP-binding protein)" |
| TTTTTCAGCAAATTT | thiC | thiamine-phosphate pyrophosphorylase |
| TTTTTCAGACAATTG | yvaV | similar to hypothetical proteins |
| ATTGTCAGCATATTT | fliR | required for flagellar formation |
| TTTTTCATAAAATTT | yokA | assimilatory nitrate reductase |
| TTTTTCATAAAATTC | yqeY | similar to hypothetical proteins |
| ATTGTTAGAAAATTA | yjcL | function="unknown" |
| ACTTTCAGAATATTT | rapE | response regulator aspartate phosphatase" |
| TTTTTCTGAATAATT | bfmBB | branched-chain alpha-keto acid dehydrogenase E2 subunit |

| | | |
|---|---|---|
| Detected motifs are sorted according to their similarity to the consensus | | |

**Table 7. Whole genome search for the presence of the putative CodY box in intergenic regions of Streptococcus pneumoniae.**

| **Sequence** | **Gene** | **Description** |
|---|---|---|
| TGATTTTCAGAAAAATTTAAGAAAAA | ppmA | Proteinase maturation protein |
| ATATTTTCTGAAAATTTCTTCAGTAA | Leu-A | 2-isopropylmalate synthase, truncation |
| TTATTATCAGATAATTTTATCAATCG | hsds | type I restriction enzyme |
| CAATTTTCTGATAATTCGGTATATTC | livJ | ABC transporter branched chain amino acid |
| GAATTTTCTGAAAATTACAAAATATA | ilvE | Branched-chain-amino-acid transaminase |
| TTATTTTCTGAAAATTTGGTAAAATA | gapN | NADP-dependent glyceraldehyde-3-phosphate |
| TCATTTTTTGAAAAAATGATTATTAC | mefE | ABC transporter - macrolide efflux |
| CATTTTTCAGAAAATTCTTTTATTTC | IS1381 | Degenerate transposase |
| ACATTTTCTGAAATTAAAAATAATAT | ugd | UDP-glucose dehydrogenase |
| GAAATTTCTGAAAAATATGATATAAT | hemK | protoporphyrinogen oxidase |
| AAATTATTTGATAATTCTATAATTTC | spr1649 | Putative transcriptional regulator (phoU like) |
| AATTTATCTGAAAAAACGAAAAATAT | spr1765 | Hypothetical protein |
| CAATTTTTTGAAAAAATATTGATTTA | glgB | 1,4-alpha-glucan branching enzyme |
| TATTTTTCTGAAAACTCTGATATAAA | spr2010 | Probable member of DHH superfamily |
| TCATTTTCAGATAAGGATAAAAATTG | spr0128 | Hypothetical protein |
| GAATTGACAGATGAATTTGTTAAGAA | ABC-NDB | ABC transporter ATP-binding protein - unknown |
| AAATTGAATGAAAAGTATAAAATTAA | spr1149 | Probable oligosaccharide repeat unit transporter |
| AAATTGTCAGAATTATGAGAAAATAG | rgg/spr1934 | transcriptional regulator of glucosyltransferase |
| CATTTTACTGAAGAATACGATATTAT | gidA | Glucose inhibited division protein |
| AAATTTCTAGATAAGTTAATAATTAA | abc-n/p | ABC transporter - unknown substrate |
| ATATTCTGTGAAAAATAAAATAGTAT | spr0119 | Hypothetical protein |
| GAATTTCCAGATAAACTAAAAAAATC | hsdS 2X | type I restriction enzyme |
| ACATTATCTGAAAAATTAAACTATAA | spr0607 | Hypothetical protein |
| TAATTGTCAGAAAGTTAAATAAAGGA | rpmI | 50S Ribosomal protein L35 |
| AAATTATCTAATAACAAAAATATTAT | spnIM | DNA modification methyltransferase |
| TTATTTACAGAAAGAACAAAAAATGC | spnIM | DNA modification methyltransferase |
| GAATTTTCAGAAAATTCTATACGCAT | ilvD | Dihydroxyacid dehydratase |
| ATATTTTTTGAAAAATTTTTTAAAAA | arcA | Arginine deiminase, truncation |
| AAATTGACAAATAAAAATTTGAATAT | ABC-NBD | ABC transporter ATP-binding protein anion transport |
| TACTTTACAGAAGAATTACAGAATAG | mfd | Transcription-repair coupling factor |
| GAATTTTCAGAATAATCTGTATATGT | spr0157 | Conserved hypothetical protein |
| GAATTTCCTGAAAATCTGGCTATTAT | murM | Serine/alanine adding enzyme |
| GATTTTTCGGAAAATTATGTTAGAAT | zwf | Glucose-6-phosphate 1-dehydrogenase |
| CAATATTTAGAAAAAAAAGAAATTAA | licD1 | phosphorylcholine incorporation in teichoic acids |
| ATATTTTCTGAACAATTAATATATTC | spr1403 | Hypothetical protein |
| CATTTTGCGGAAAATTGAGTAAATAT | relA | GTP pyrophosphokinase |
| TAATTTTCTGATTTTTTGTAAAATAA | spr1650 | Hypothetical protein |
| TTATTTTCTAATAGATATAAAATTAT | pcpA | Choline-binding protein |
| TAAATTTCAGATTGATGAAAAAATAG | rsuA | Ribosomal small subunit pseudouridine synthase |
| AATTTTTTAGAAAAAAGTGTAATTTT | ptsG | PTS glucose-specific enzyme IIABC component |
| AAATTCACTGAAAGTTTAAATATGAC | hsdS | type I restriction enzyme |
| GAATTGCCAGACTATTTTAATACTAT | spr0803 | Hypothetical protein |
| AGATTTTAAGTAAAATTTATTAGTAA | spr0826 | Hypothetical protein |
| AAATTTTAGAAAAAATTAAAGAATAC | flaV | Flavodoxin |
| CAATTATCTGATCATCTGAAAAATAT | ABC-NP | ABC transporter - multidrug resistance |

| | | |
|---|---|---|
| Detected motifs are sorted according to their similarity to the consensus | | |

**Table 8. Whole genome search for the presence of the putative CodY box in intergenic regions of Streptococcus agalacticiae.**

| **Sequence motif** | **Gene** | **Description** |
|---|---|---|
| ATTATCAGAATATTG | gbs 1632 | similar to branched-chain amino acid ABC transporter |
| ATTTTCAGAAAAATA | gbs1105 | similar to unknown protein |
| TTTATCAGAAAATTT | gbs0662 | similar to ABC transporter (ATP-binding protein) |
| ATTTTCTGAATATTC | gbs2002 | similar to glycerol dehydrogenase |
| TTTTTCTGAATATTT | gbs1489 | possible surface protein |
| ATTTTCAAAAAATTT | gbs0054 | similar to alcohol dehydrogenase |
| ATTTTCAAAAAATTG | gbs0008 | similar to unknown protein |
| ATTGTCAGAATTTTC | gbs1406 | Similar to ABC transporter |
| ATTATCTGAAAATTT | gbs0144 | similar to oligopeptide ABC transporter |
| ATTATCTGAATATTA | gbs0898 | acetoin dehydrogenase E3 |
| ATTTTCAGTATATTC | gbs2007 | similar to C5A peptidase, putative peptidoglycan linked protein |
| ATTTTCAGAAAATGT | gbs0235 | gene="rRNA-16s |
| ATTATCAGAAGATTT | gbs0577 | Similar to unknown proteins |
| ATTTTCAGATAATTG | gbs0143 | similar to oligopeptide ABC transporter |
| ATTTTTAGAAAATTA | gbs0604 | similar to negative regulator of FtsZ ring formation protein EzrA |
| ATTTTCTGAATAATT | dnaG | DNA primase |
| ATTTTCTGAATAATT | ftsA | Similar to cell division protein FtsA |
| ATTTTCAGGATATTT | gbs1259 | Similar to ABC transporter |

| | | |
|---|---|---|
| Detected motifs are sorted according to their similarity to the consensus | | |

**Table 9. Bacterial strains and plasmids used in this patent application**

| Strain or plasmid | Relevant phenotype or genotype | Source or reference |
|---|---|---|
| Strains | | |
| *L. lactis* subsp. *cremoris* | | |
| MG1363 | Lac⁻; Prt⁻; Plasmid-free derivative of NCDO712 | (Gasson, 1983) |
| LL108 | Cm^{r}, MG1363 derivative containing pWV01 *repA* gene in the chromosome | (Leenhouts *et al.,* 1998) |
| LL302 | RepA⁺ MG1363, carrying one copy of pWV01 *repA* gene on the chromosome | (Leenhouts *et al*., 1998) |
| NZ9000 | MG1363 *pepN::nisRK* | (Kuipers *et al*., 1998) |
| NZ9700 | Nisin producing transconjugant containing the nisin-sucrose transposon Tn5276 | (Kuipers *et al*., 1993) |
| SK11 | Lac⁺; Prt⁺; harbours, a.o., proteinase plasmid pSK111 | (de Vos *et al*., 1984) |
| Wg2 | Prt⁺; harbours, a.o., proteinase plasmid pWV05 | (Otto *et al.,* 1982) |

| *L. lactis* subsp. *lactis* | Lac⁺; Prt⁺ | (Kok, 1990) |
|---|---|---|
| BGMN1-5 *E. coli* | Wild type strain, PrtP⁺, Bac501⁺, Bac513⁺ | (Gajic *et al*., 1999) |
| EC101 | Kan^{r}; JM101 with *repA* from pWV01 integrated in chromosome | J.Law *et al*., 1995 |
| Plasmids pNZ8048 | Cm^{r}, inducible expression vector carrying P_{nisA} | (Kuipers *et al*., 1998) |
| pNH6CodY | *his6-codY* of *L. lactis* MG1363 behind P_{nisA} | Gajić, to be submitted |
| pGKH11 | Em^{r}, Cm^{r}, contains genes for α-gal and β-gal, controlled by P_{rtM} and P_{prtP} of Wg2, respectively | (Haandrikman, 1990) |
| pGKB11 | Em^{r}, Cm^{r}, contains genes for α-gal and β-gal, controlled by P_{prtM} and P_{prtP} of BGMN1-5, respectively | Gajić, to be submitted |
| pGKE11 | Em^{r}, Cm^{r}, contains genes for α-gal and β-gal, controlled by P_{prtM} and P_{prtP} of E8, respectively | Gajić, to be submitted |
| pGKS11 | Em^{r}, Cm^{r}, contains genes for α-gal and β-gal, controlled by P_{prtM} and P_{prtP} of SK11, respectively | Gajić, to be submitted |
| pORI13 | Em^{r}; integration vector, promoterless *lacZ,* Ori⁺, RepA- derivative of pWV01 | J. Sanders *et al*., 1998 |
| pORIopp14 | Em^{r}; pORI13 carrying 154bp *oppD* promoter fragment amplified with primers opp1 and opp 14 | Gajić, to be submitted |
| pORIopp15 (a) | Em^{r}; pORI13 carrying 154bp *oppD* promoter fragment amplified with primers opp1 and opp15(a) | Gajić, to be submitted |
| pORIopp15 (b) | Em^{r}; pORI13 carrying 154bp *oppD* promoter fragment amplified with primers opp1 and opp15(b) | Gajić, to be submitted |

| | | |
|---|---|---|
| Em^{r}, Cm^{r} Ap^{r}, resistance to erythromycin, chloramphenicol and ampicilin, respectively. P_{nisA}-inducible *nisA* promoter, P_{prtP} - *_{PrtP}* promoter, P_{prtM} - *_{PrtM}* promoter, α-gal - α-galactosidase, β-gal - β-galactosidase. | | |

**Table 10. Differentially expressed genes found in microarray expression analysis of L. lactis MG1363 codY vs. WT. (NA = not available)**

| Gene | Average regulation (fold in *codY vs.* WT) | Significance (*p*-value) |
|---|---|---|
| opts | 9.8 | 7.17E-05 |
| gltD | 7.3 | 2.18E-05 |
| citB | 7.1 | 3.76E-03 |
| asnB | 6.0 | 1.08E-05 |
| ctrA | 5.8 | 3.68E-05 |
| ilvD | 5.4 | 3.81E-05 |
| pepO | 5.0 | 1.93E-05 |
| ymdC | 4.9 | 5.29E-04 |
| hisA | 4.9 | 2.52E-06 |
| ilvN | 4.7 | 6.10E-03 |
| oppA | 4.6 | 1.13E-05 |
| icd | 3.1 | 1.25E-05 |
| oppD | 3.1 | 5.86E-05 |
| hisB | 3.0 | 1.12E-02 |
| leuD | 3.0 | 5.09E-01 |
| udp | 3.0 | 1.89E-03 |
| oppB | 2.8 | 1.69E-04 |
| yahD | 2.7 | 1.15E-01 |
| oppC | 2.7 | 1.67E-03 |
| yohC | 2.6 | 3.00E-02 |
| yveA | 2.6 NA | |
| uxuA | 2.5 NA | |
| serC | 2.5 | 2.49E-03 |
| oppF | 2.5 | 2.92E-06 |
| trxA | 2.4 NA | |
| arcC2 | 2.3 | 5.03E-03 |
| ilvC | 2.3 | 9.65E-02 |
| serA | 2.3 | 2.86E-02 |
| argG | 2.3 | 2.29E-02 |
| arcD1 | 2.3 | 1.80E-04 |
| hisK | 2.2 | 7.72E-02 |
| yxbA | 2.2 NA | |
| ydgD | 2.2 NA | |
| serB | 2.2 | 7.60E-04 |
| leuC | 2.1 NA | |
| optA | 2.1 NA | |
| yjjA | 2.1 | 4.31E-01 |
| asnS | 2.1 | 4.31E-02 |
| hisl | 2.1 | 8.82E-05 |
| yxaC | 2.1 | 9.81E-02 |
| groES | 2.1 | 2.40E-03 |
| yjhC | 2.0 | 1.75E-01 |
| rmeC | 2.0 | 1.49E-01 |
| ytdB | 2.0 | 2.31E-01 |
| ysiC | 2.0 NA | |
| yndG | 2.0 | 1.36E-01 |
| optD | 2.0 | 4.14E-02 |
| a mtB | 2.0 | 1.67E-04 |
| feoB | 1.9 NA | |
| hisH | 1.9 | 2.25E-02 |
| ykcC | 1.9 NA | |
| lysA | 1.9 | 1.63E-03 |
| pepN | 1.9 | 1.29E-03 |
| glgC | 1.9 | 3.76E-02 |
| aldB | 1.9 | 5.52E-05 |
| areC1 | 1.8 NA | |
| lcnD | 1.8 NA | |
| hrcA | 1.8 | 1.48E-03 |
| yfiG | 1.8 | 1.27E-01 |
| thrC | 1.7 | 1.30E-02 |
| ydiE | 1.7 | 2.15E-01 |
| ilvA | 1.7 NA | |
| yxdG | 1.7 | 1.09E-02 |
| ynaD | 1.7 | 1.08E-03 |
| yedE | 1.7 | 2.28E-01 |
| thrA | 1.7 | 1.86E-02 |
| rodA | 1.7 | 1.18E-01 |
| ydiG | 1.7 | 1.29E-01 |
| yfiD | 1.7 | 2.82E-01 |
| yhfE | 1.7 | 7.89E-02 |
| yahA | 1.7 | 1.30E-01 |
| ydcG | 1.7 | 1.27E-04 |
| gltA | 1.6 | 6.48E-02 |
| yviC | 1.6 | 1.51E-01 |
| ybaD | 1.6 | 1.02E-01 |
| ybjA | 1.6 | 2.65E-01 |
| hisD | 1.6 | 2.76E-01 |
| glk | 1.6 | 7.18E-02 |
| rgpE | 1.6 | 2.74E-01 |
| thrB | 1.6 | 1.25E-01 |
| clpB | 1.6 NA | |
| bcaT | 1.6 | 3.87E-03 |
| hisF | 1.6 NA | |
| yvcA | 1.6 | 3.16E-01 |
| ymgK | 1.6 NA | |
| yajH | 1.6 | 3.50E-01 |
| yccE | 1.6 | 1.49E-01 |
| yndA | 1.6 | 4.79E-04 |
| ybhE | 1.6 | 1.43E-01 |
| adhE | 1.6 | 1.53E-02 |
| grpE | 1.6 | 4.25E-02 |
| yqaB | 1.5 | 2.78E-03 |
| pgiA | 1.5 | 1.19E-01 |
| yrh H | 1.5 NA | |
| ybhC | 1.5 | 2.97E-01 |
| glgD | 1.5 | 1.57E-02 |
| yidA | 1.5 | 1.13E-01 |
| yabF | 1.5 | 1.54E-02 |
| rimM | 1.5 | 1.44E-01 |
| yacl | 1.5 | 2.36E-01 |
| asd | 1.5 NA | |
| yijE | 1.5 | 2.02E-01 |
| yccF | 1.5 | 6.44E-02 |
| agl | 1.5 | 1.28E-01 |
| optC | 1.5 | 1.91E-02 |
| malF | 1.5 | 8.17E-02 |
| yjhB | 1.5 | 4.32E-01 |
| yibG | 1.5 | 4.78E-04 |
| ribA | 1.5 NA | |
| yabE | 1.5 | 3.23E-01 |
| thiL | 1.5 | 2.06E-01 |
| ycaF | 1.5 | 7.56E-02 |
| optF | 1.5 | 5.78E-02 |
| arcC3 | 1.5 NA | |
| ymfD | 1.5 | 2.40E-01 |
| ps309 | 1.5 | 2.41E-01 |
| ycfl | 1.5 | 1.68E-01 |
| uvrC | 1.5 | 5.04E-01 |
| yphC | 1.5 | 2.67E-01 |
| ybeM | 1.5 | 2.42E-01 |
| ygfA | 1.5 NA | |
| yphJ | 1.5 | 1.00E-01 |
| ynaA | 1.5 | 9.20E-02 |
| ahpF | 1.5 | 1.75E-01 |
| aroB | 1.5 | 1.47E-01 |
| ybhD | 1.5 | 1.85E-01 |
| hisC | 1.5 | 6.57E-02 |
| yoaF | 1.5 | 3.16E-01 |
| dexC | 1.5 | 2.59E-02 |
| amyY | 1.5 | 3.85E-02 |
| yueF | 1.5 | 5.47E-01 |
| ydgH | 1.5 | 4.84E-01 |
| dapB | 1.4 | 1.11E-03 |
| ftsZ | 1.4 | 1.35E-01 |
| ynhA | 1.4 | 1.07E-02 |
| ygiJ | 1.4 | 2.11E-01 |
| ptcA | 1.4 | 1.29E-01 |
| yqeH | 1.4 | 2.45E-02 |
| yrgl | 1.4 | 6.06E-01 |
| pepC | 1.4 | 2.97E-03 |
| ycfH | 1.4 | 4.66E-01 |
| ybcH | 1.4 | 4.45E-02 |
| pi345 | 1.4 | 3.31E-01 |
| ps112 | 1.4 | 5.32E-01 |
| yteD | 1.4 NA | |
| ythB | 1.4 | 9.95E-02 |
| yneD | 1.4 | 7.17E-02 |
| pmsR | 1.4 | 7.19E-02 |
| rgpC | 1.4 | 1.34E-01 |
| yjbB | 1.4 | 3.70E-01 |
| ygdF | 1.4 | 2.48E-01 |
| yoaB | 1.4 | 1.38E-01 |
| yfhK | 1.4 | 1.10E-02 |
| yvaB | 1.4 | 4.18E-01 |
| yqjA | 1.4 | 1.45E-01 |
| pi120 | 1.4 | 1.27E-03 |
| ylic | 1.4 | 6.76E-02 |
| ydgC | 1.4 | 5.49E-04 |
| rarA | 1.4 | 1.18E-01 |
| ywdG | 1.4 | 1.11E-01 |
| yfdD | 1.4 | 2.09E-01 |
| ftsW1 | 1.4 | 6.18E-03 |
| ybeB | 1.4 | 7.74E-02 |
| yvdD | 1.4 | 9.09E-03 |
| ymeB | 1.4 | 2.19E-02 |
| yoiC | 1.4 NA | |
| yhcK | 1.4 | 3.80E-01 |
| groEL | 1.4 NA | |
| xylM | 1.4 | 1.38E-01 |
| ydgG | 1.4 | 3.77E-01 |
| sugE | 1.4 | 1.86E-01 |
| pi360 | 1.4 | 3.37E-01 |
| yfhB | 1.4 | 2.97E-01 |
| metS | 1.4 | 6.97E-02 |
| yeaC | 1.4 NA | |
| coiA | 1.4 | 1.76E-01 |
| butA | 1.4 | 5.62E-03 |
| clpP | 1.3 | 1.82E-01 |
| arcA | 1.3 | 1.20E-01 |
| yacB | 1.3 | 4.34E-02 |
| kinC | 1.3 | 2.34E-02 |
| ps305 | 1.3 | 2.30E-01 |
| glnB | 1.3 | 2.92E-01 |
| ybbC | 1.3 | 3.99E-02 |
| ykhJ | 1.3 | 8.11E-02 |
| dnaK | 1.3 | 9.17E-02 |
| dnaG | 1.3 | 2.16E-03 |
| ybdJ | 1.3 | 3.38E-01 |
| yohD | 1.3 | 1.39E-04 |
| pi130 | 1.3 NA | |
| yugC | 1.3 | 1.75E-03 |
| ycjH | 1.3 | 3.25E-01 |
| yjaB | 1.3 | 1.33E-01 |
| glgP | 1.3 | 7.93E-02 |
| pi235 | 1.3 NA | |
| acmB | 1.3 | 1.78E-01 |
| xylX | 1.3 NA | |
| ybeH | 1.3 | 1.00E-01 |
| arcD2 | 1.3 | 1.82E-03 |
| rgpB | 1.3 | 6.26E-02 |
| asnH | 1.3 | 2.29E-01 |
| yedF | 1.3 | 5.47E-02 |
| accB | 1.3 | 5.35E-02 |
| fhuD | 1.3 | 5.56E-02 |
| yccG | 1.3 | 1.56E-01 |
| ypiH | 1.3 | 2.11E-01 |
| yacc | 1.3 | 2.26E-01 |
| ybeF | 1.3 | 2.13E-01 |
| ygaE | 1.3 | 3.66E-01 |
| ygaF | 1.3 | 1.38E-01 |
| ybeD | 1.3 | 2.18E-01 |
| pi302 | 1.3 NA | |
| ps302 | 1.3 NA | |
| ydgB | 1.3 | 1.14E-03 |
| hemK | 1.3 | 6.48E-02 |
| ychH | 1.3 | 2.07E-03 |
| yjhD | 1.3 | 6.68E-03 |
| yfjA | 1.3 | 2.97E-02 |
| ykhD | 1.3 | 1.11E-02 |
| accC | 1.3 | 1.54E-01 |
| ynaC | 1.3 | 1.95E-02 |
| cdsA | 1.3 | 1.94E-01 |
| mtsA | 1.3 | 2.11E-02 |
| murA2 | 1.3 | 1.46E-01 |
| ps120 | 1.3 | 8.81E-02 |
| yejJ | 1.3 | 1.31E-01 |
| ybhB | 1.3 | 2.25E-01 |
| ysaA | 1.3 | 3.07E-01 |
| yagA | 1.3 | 2.96E-01 |
| pi336 | 1.3 | 1.09E-01 |
| yjjD | 1.3 | 3.11E-01 |
| yjcA | 1.3 | 1.25E-02 |
| IIrA | 1.3 | 2.06E-01 |
| msmK | 1.3 | 2.57E-01 |
| ybdA | 1.3 | 3.20E-01 |
| yjhF | 1.3 | 5.21E-04 |
| yrbC | 1.3 | 3.38E-01 |
| yhfF | 1.3 | 1.43E-02 |
| yjdE | 1.3 | 1.95E-02 |
| yhcA | 1.3 | 1.56E-01 |
| yjiB | 1.3 | 1.68E-01 |
| yjjC | 1.3 | 3.30E-02 |
| ycfG | 1.3 | 2.08E-01 |
| ycdF | 1.3 | 4.78E-01 |
| ymhC | 1.3 | 4.73E-01 |
| yjcF | 1.3 | 2.48E-01 |
| ps122 | 1.3 | 4.37E-02 |
| ywcC | 1.3 | 3.77E-01 |
| xylT | 1.3 | 8.99E-02 |
| araT | 1.3 | 1.20E-02 |
| ycjG | 1.3 | 5.87E-02 |
| yjgF | 1.3 | 3.49E-01 |
| clpE | 1.3 | 2.05E-01 |
| frdC | 1.3 | 5.17E-03 |
| gpdA | 1.3 | 9.30E-02 |
| ychD | 1.3 | 1.91E-01 |
| yniJ | 1.3 | 1.91E-02 |
| glnP | 1.3 | 1.65E-01 |
| butB | 1.3 | 1.31E-01 |
| pyrG | 1.3 | 2.66E-01 |
| ftsW2 | 1.3 | 1.21E-01 |
| yhhC | 1.3 NA | |
| noxD | 1.3 | 5.88E-03 |
| dinP | 1.3 NA | |
| recQ | -1.3 | 1.59E-01 |
| lplL | -1.3 | 9.88E-03 |
| xynD | -1.3 | 1.04E-01 |
| pi225 | -1.3 | 3.54E-01 |
| yjfG | -1.3 | 4.16E-02 |
| rheB | -1.3 | 2.17E-01 |
| pi140 | -1.3 | 2.73E-01 |
| rpmGC | -1.3 | 1.58E-01 |
| yxbC | -1.3 | 1.51E-02 |
| bglR | -1.3 | 2.45E-01 |
| yqbl | -1.3 | 5.59E-01 |
| yghB | -1.3 | 6.78E-02 |
| yqcA | -1.3 | 4.10E-01 |
| ynjE | -1.3 | 1.85E-02 |
| ymbK | -1.3 | 1.67E-01 |
| ysjD | -1.3 | 3.18E-01 |
| yweC | -1.3 | 2.43E-02 |
| galM | -1.3 | 3.24E-01 |
| ywfC | -1.3 | 2.66E-01 |
| ps201 | -1.3 | 3.98E-01 |
| pi216 | -1.3 | 3.19E-01 |
| ykbC | -1.3 | 1.31E-01 |
| yeeB | -1.3 | 9.29E-03 |
| ileS | -1.3 | 2.32E-01 |
| yjfB | -1.3 NA | |
| yxeA | -1.3 | 2.88E-03 |
| yciF | -1.3 | 1.57E-02 |
| dukA | -1.3 | 1.34E-02 |
| ywjC | -1.3 NA | |
| yvdA | -1.3 | 8.07E-02 |
| alaS | -1.3 | 1.01E-01 |
| ndrH | -1.3 | 3.45E-03 |
| gadR | -1.3 | 2.05E-01 |
| yohH | -1.3 | 8.77E-02 |
| ps119 | -1.3 | 1.74E-01 |
| ycgJ | -1.3 | 3.16E-02 |
| pi223 | -1.3 | 1.94E-01 |
| yveD | -1.3 | 3.95E-01 |
| sunL | -1.3 | 4.87E-01 |
| yjbE | -1.3 | 1.40E-02 |
| ywbB | -1.3 | 5.42E-01 |
| yteC | -1.3 | 1.24E-01 |
| metE | -1.3 | 2.85E-01 |
| ycbD | -1.3 | 5.14E-03 |
| purN | -1.3 | 2.65E-01 |
| pabB | -1.3 | 6.63E-02 |
| yrgF | -1.3 | 1.22E-01 |
| ycjM | -1.3 | 1.23E-01 |
| yljC | -1.3 | 4.16E-01 |
| yjeF | -1.3 | 1.21E-01 |
| comGC | -1.3 | 2.23E-01 |
| yvel | -1.3 | 6.03E-02 |
| ylcD | -1.3 | 3.25E-01 |
| dltD | -1.3 NA | |
| pi343 | -1.3 | 5.49E-01 |
| yueD | -1.3 NA | |
| ppiA | -1.3 | 1.43E-01 |
| dut | -1.3 | 1.00E-01 |
| purB | -1.3 | 6.79E-03 |
| ypjC | -1.3 | 3.96E-01 |
| ytaB | -1.3 NA | |
| cbr | -1.3 | 2.49E-01 |
| rpmC | -1.3 | 3.99E-01 |
| ycgH | -1.3 | 4.57E-01 |
| ybiE | -1.3 | 2.75E-04 |
| yvdF | -1.3 | 1.24E-01 |
| rpmD | -1.3 | 2.07E-03 |
| pi124 | -1.3 | 5.76E-01 |
| pbp2B | -1.3 | 2.74E-01 |
| ypjA | -1.3 | 1.21E-01 |
| yscE | -1.3 | 4.69E-01 |
| ymbC | -1.3 | 2.22E-02 |
| birA1 | -1.4 | 1.24E-02 |
| hflX | -1.4 | 1.48E-02 |
| ffh | -1.4 | 1.60E-01 |
| yecE | -1.4 | 1.13E-01 |
| ycjl | -1.4 | 1.31E-01 |
| ps315 | -1.4 | 6.96E-02 |
| comX | -1.4 | 2.55E-01 |
| pi215 | -1.4 | 1.81E-01 |
| dcdA | -1.4 | 2.51E-02 |
| glyA | -1.4 | 1.19E-01 |
| yueA | -1.4 | 1.22E-04 |
| hsdM | -1.4 | 1.36E-01 |
| ybiD | -1.4 | 6.02E-03 |
| queA | -1.4 | 6.69E-03 |
| ywaD | -1.4 | 4.66E-01 |
| pi123 | -1.4 NA | |
| pi308 | -1.4 | 1.56E-01 |
| phnC | -1.4 | 1.79E-01 |
| ycjA | -1.4 | 3.16E-02 |
| ypjl | -1.4 | 1.99E-01 |
| ykhG | -1.4 | 4.29E-02 |
| dnaA | -1.4 | 8.81E-03 |
| phnE | -1.4 | 7.20E-03 |
| yudD | -1.4 | 5.29E-01 |
| yrfA | -1.4 | 2.43E-01 |
| yeiD | -1.4 | 2.64E-03 |
| rpmF | -1.4 | 2.56E-02 |
| ysfG | -1.4 | 2.86E-01 |
| pi249 | -1.4 | 1.60E-01 |
| yuiB | -1.4 | 3.97E-01 |
| yvaD | -1.4 | 9.44E-02 |
| reeD | -1.4 | 1.42E-01 |
| yfcB | -1.4 | 1.72E-01 |
| kdgR | -1.4 | 7.74E-01 |
| ps304 | -1.4 | 4.41E-02 |
| yqfC | -1.4 NA | |
| yxbF | -1.4 | 8.14E-05 |
| citE | -1.4 | 5.93E-02 |
| yfcC | -1.4 | 2. 53E-01 |
| folD | -1.4 | 9.71E-02 |
| pdhD | -1.4 | 2.62E-03 |
| ps118 | -1.4 | 1.75E-01 |
| yafF | -1.4 | 3.14E-02 |
| nagB | -1.5 | 6.51E-04 |
| ymcF | -1.5 | 5.55E-01 |
| yveB | -1.5 NA | |
| yxbD | -1.5 | 3.56E-02 |
| pi319 | -1.5 | 6.45E-02 |
| yoaD | -1.5 | 3.74E-01 |
| ywaH | -1.5 | 4.10E-01 |
| gidC | -1.5 | 9.87E-03 |
| yfbK | -1.5 | 6.66E-03 |
| yrgA | -1.5 | 2.34E-01 |
| mgtA | -1.5 | 1.06E-02 |
| ynbE | -1.5 | 2.98E-01 |
| yrbE | -1.5 | 6.75E-02 |
| ynfH | -1.5 | 3.06E-02 |
| purE | -1.5 | 4.33E-01 |
| ytjE | -1.5 | 2.12E-01 |
| ymgF | -1.5 NA | |
| gltS | -1.5 | 1.23E-01 |
| kdgK | -1.5 | 3.45E-01 |
| yccH | -1.5 NA | |
| kinB | -1.5 | 1.09E-01 |
| yuiA | -1.5 | 6.52E-02 |
| yxbE | -1.6 | 4.59E-04 |
| yceE | -1.6 | 4.59E-02 |
| yueE | -1.6 | 5.60E-03 |
| ywdB | -1.6 | 3.02E-01 |
| zitR | -1.6 | 2.11E-01 |
| ypdB | -1.6 | 1.75E-01 |
| yjgB | -1.6 | 3.73E-02 |
| argD | -1.6 | 1.74E-01 |
| ps113 | -1.6 | 2.75E-01 |
| purK | -1.6 NA | |
| yrbB | -1.6 | 4.82E-02 |
| yjgC | -1.7 | 8.92E-02 |
| ykiC | -1.7 | 2.54E-01 |
| purH | -1.7 | 1.66E-01 |
| rmaB | -1.7 NA | |
| cysD | -1.7 | 3.29E-01 |
| nrdG | -1.7 | 5.10E-03 |
| pi104 | -1.7 | 1.19E-01 |
| yscD | -1.7 | 3.63E-01 |
| gntR | -1.7 | 1.82E-01 |
| tra983L | -1.7 | 3.08E-01 |
| gntK | -1.7 | 4.13E-03 |
| dxsA | -1.7 NA | |
| yjal | -1.7 | 1.15E-01 |
| ynjB | -1.7 NA | |
| rmaC | -1.7 | 1.79E-01 |
| pi230 | -1.8 | 2.01E-01 |
| yriD | -1.8 | 2.06E-01 |
| yhcE | -1.8 | 2.70E-03 |
| yveF | -1.8 | 1.91 E-01 |
| ytbC | -1.8 | 2.96E-01 |
| ImrA | -1.8 | 3.06E-01 |
| yhhA | -1.8 NA | |
| purM | -1.9 | 1.99E-01 |
| codY | -1.9 | 1.04E-02 |
| pi122 | -1.9 NA | |
| yiiD | -1.9 | 2.86E-01 |
| argH | -1.9 | 8.77E-02 |
| yhcH | -2.0 | 3.61 E-01 |
| panE | -2.0 | 9.68E-03 |
| purF | -2.0 | 5.26E-03 |
| ps206 | -2.0 NA | |
| metB2 | -2.0 | 5.73E-02 |
| guaC | -2.0 | 1.40E-01 |
| cysM | -2.0 | 1.88E-02 |
| yviH | -2.1 | 1.80E-01 |
| uxaC | -2.1 | 2.96E-01 |
| gmk | -2.1 NA | |
| rpsU | -2.1 NA | |
| ycgA | -2.2 NA | |
| cysK | -2.2 | 7.17E-03 |
| yshA | -2.3 | 1.71 E-03 |
| ymgH | -2.3 | 2.11 E-01 |
| llrG | -2.3 NA | |
| ps111 | -2.4 NA | |
| pi353 | -2.5 | 2.00E-02 |
| yliG | -2.5 | 2.61 E-01 |
| ynjJ | -2.5 | 1.82 E-01 |
| yndE | -2.6 NA | |
| hpt | -2.6 NA | |
| yojC | -2.7 NA | |
| plpC | -2.7 | 5.16E-03 |
| plpD | -2.7 | 1.97E-03 |
| ywdA | -2.8 | 2.03E-01 |
| yviJ | -3.2 NA | |
| purC | -3.3 NA | |
| plpB | -3.4 | 1.53E-03 |
| purL | -3.4 | 5.25E-02 |
| plpA | -3.4 | 1.08E-03 |

### REFERENCES

Alvarez,D., Novac,O., Callejo,M., Ruiz,M.T., Price,G.B., and Zannis-Hadjopoulos,M. (2002) 14-3-3sigma is a cruciform DNA binding protein and associates in vivo with origins of DNA replication J.Cell Biochem. 87: 194-207.
Buist, G., H. Karsens, A. Nauta, D. van Sinderen, G. Venema, and J. Kok. 1997. Autolysis of Lactococcus lactis caused by induced overproduction of its major autolysin, AcmA. Appl.Environ.Microbiol 63:2722-2728.
Christensen, J. E., Dudley, E. G., Pederson, J. A. and Steele, J. L. (1999). Peptidases and amino acid catabolism in lactic acid bacteria. Antonie Van Leeuwenhoek 76 , 217-246.
Detmers, F. J., Kunji, E. R., Lanfermeijer, F. C., Poolman, B. and Konings, W. N. (1998). Kinetics and specificity of peptide uptake by the oligopeptide transport system of Lactococcus lactis. Biochemistry 37, 16671-16679.
Ebbole, D. J. and Zalkin, H. (1989). Interaction of a putative repressor protein with an extended control region of the Bacillus subtilis pur operon. J.Biol. Chem. 264, 3553-3561.
Exterkate, F. A. (1985). A dual directed control of cell wall proteinase production in S. cremoris AM1: a possible mechanism of regulation during growth in milk. Journal of Dairy Science 68, 562-571.
Fisher,S.H., Rohrer,K., and Ferson,A.E. (1996) Role of CodY in regulation of the Bacillus subtilis hut operon J.Bacteriol. 178: 3779-3784.
Foucaud, C., Kunji, E. R., Hagting, A., Richard, J., Konings, W. N., Desmazeaud, M. and Poolman, B. (1995). Specificity of peptide transport systems in Lactococcus lactis: evidence for a third system which transports hydrophobic di- and tripeptides. J.Bacteriol. 177, 4652-4657.
Gajic, O., Kojic, M., Banina, A. and Topisirovic, L. (1999). Characterization of natural isolate Lactococcus lactis subsp. lactis BGMN1-5, a strain producing two bacteriocins, cell wall-associated proteinase and showing clumping phenotype. Arch.Biol.Sci.Belgrade 51, 69-78.
Gasson, M. J. (1983). Plasmid complements of Streptococcus lactis NCDO 712 and other lactic streptococci after protoplast-induced curing. J.Bacteriol. 154, 1-9.
Guedon,E., Renault,P., Ehrlich,S.D., and Delorme,C. (2001a) Transcriptional pattern of genes coding for the proteolytic system of Lactococcus lactis and evidence for coordinated regulation of key enzymes by peptide supply J.Bacteriol. 183: 3614-3622.
Guedon,E., Serror,P., Ehrlich,S.D., Renault,P., and Delorme,C. (2001b) Pleiotropic transcriptional repressor CodY senses the intracellular pool of branched-chain amino acids in Lactococcus lactis Mol.Microbiol. 40: 1227-1239.
Haandrikman, A. J. (1990). Maturation of the cell envelope-associated proteinase of Lactococcus lactis. 64-71.
Hamoen, L. W., A. F. Van Werkhoven, J. J. Bijlsma, D. Dubnau, and G. Venema. 1998. The competence transcription factor of Bacillus subtilis recognizes short A/T-rich sequences arranged in a unique, flexible pattern along the DNA helix. Genes Dev. 12:1539- 1550.
Israelsen, H., S. M. Madsen, A. Vrang, E. B. Hansen, and E. Johansen. 1995. Cloning and partial characterization of regulated promoters from Lactococcus lactic Tn917-lacZ integrants with the new promoter probe vector, pAK80. Appl.Environ.Microbiol. 61:2540- 2547.
Kiwaki, M., Ikemura, H., Shimizu-Kadota, M. and Hirashima, A. (1989b). Molecular characterization of a cell wall-associated proteinase gene from Streptococcus lactis NCDO763. Mol.Microbiol. 3, 359-369.
Kok, J., van Dijl, J. M., van der Vossen, J. M. and Venema, G. (1985b). Cloning and expression of a Streptococcus cremoris proteinase in Bacillus subtilis and Streptococcus lactis. Appl.Environ.Microbiol. 50, 94-101.
Kok, J., Leenhouts, K. J., Haandrikman, A. J., Ledeboer, A. M. and Venema, G. (1988a). Nucleotide sequence of the cell wall proteinase gene of Streptococcus cremoris Wg2. Appl.Environ.Microbiol. 54, 231-238.
Kok, J. (1990). Genetics of the proteolytic system of lactic acid bacteria. FEMS Microbiol.Rev. 7, 15-42.
Kok, J. and de Vos,W.M. (1993) The proteolytic system of lactic acid bacteria. London: Blackie and professional, pp. 169-210.
Kuipers, O. P., Beerthuyzen, M. M., Siezen, R. J. and de Vos, W. M. (1993b). Characterization of the nisin gene cluster nisABTCIPR of Lactococcus lactis. Requirement of expression of the nisA and nisI genes for development of immunity. Eur.J.Biochem. 216, 281-291.
Kuipers,O.P., de Ruyter,P.G., Kleerebezem,M., and Vos,W.M. (1998) Qurum sensing controlled gene expression in lactic acid bacteria Biotechnol 64: 15-21.
Kunji, E. R., Mierau, I., Hagting, A., Poolman, B. and Konings, W. N. (1996). The proteolytic systems of lactic acid bacteria. Antonie Van Leeuwenhoek 70, 187-221.
Laan, H., Bolhuis, H., Poolman, B., Abee, T. and Konings, W. N. (1993). Regulation of proteinase synthesis in Lactococcus lactis. Acta Biotechnology 13, 95-101.
Laemmli, U. K. 1970. Cleavage of structural proteins during the assembly of the head of bacteriophage T4. Nature 227:680-685.
Leenhouts, K (1995) Integration strategies and vectors. Dev. Biol. Stand. 85: 523-530.
Leenhouts,K., Buist,G., Bolhuis,A., ten Berge,A., Kiel,J., Mierau,I., Dabrowska,M., Venema,G., and Kok,J. (1996) A general system for generating unlabelled gene replacements in bacterial chromosomes Mol.Gen.Genet. 253: 217-224.
Leenhouts, K., Bolhuis, A., Venema, G. and Kok, J. (1998b). Construction of a food-grade multiple-copy integration system for Lactococcus lactis. Appl.Microbiol.Biotechnol. 49, 417-423.
Marugg, J. D., Meijer, W., van Kranenburg, R., Laverman, P., Bruinenberg, P. G. and de Vos, W. M. (1995). Medium-dependent regulation of proteinase gene expression in Lactococcus lactis: control of transcription initiation by specific dipeptides. J.Bacteriol. 177, 2982-2989.
Marugg,J.D., van Kranenburg,R., Laverman,P., Rutten,G.A., and de Vos,W.M. (1996) Identical transcriptional control of the divergently transcribed prtP and prtM genes that are required for proteinase production in lactococcus lactis SK11 J.Bacteriol. 178: 1525-1531.
Meijer, W. C., Marrug, J. D. and Hugenholtz, J. (1996). Regulation of proteolytic enzyme activity in Lactococcus lactis. Appl.Environ.Microbiol. 62, 156-161.
Mierau, L., Haandrikman, A. J., Velterop, O., Tan, P. S., Leenhouts, K. L., Konings, W. N., Venema, G. and Kok, J. (1994). Tripeptidase gene (pepT) of Lactococcus lactis: molecular cloning and nucleotide sequencing of pepT and construction of a chromosomal deletion mutant. J.Bacteriol. 176, 2854-2861.
Miladinov,N., Kuipers,O.P., and Topisirovic,L. (2001) Casitone-mediated expression of the prtP and prtM genes in Lactococcus lactis subsp. lactis BGIS29 Arch.Microbiol. 177: 54-61.
Miller, J. H. 1972. Experiments in Molecular Genetics. Cold Spring Harbor Laboratory Press, Cold Spring Harbor, NY.
Otto, R., de Vos, W. M. and Gavrieli, J. (1982). Plasmid DNA in Streptococcus cremoris Wg2: influence of pH on selection in chemostats of a varient lacking a protease plasmid. Appl.Envion.Microbiol. 43, 1272-1277.
Poolman,B., Konings,W.N. (1988) Relation of growth of Streptococcus lactis and Streptococcus cremoris to amino acid transport J.Bacteriol. 170: 700-707.
Ratnayake-Lecamwasam,M., Serror,P., Wong,K.W., and Sonenshein,A.L. (2001) Bacillus subtilis CodY represses early-stationary-phase genes by sensing GTP levels Genes Dev. 15: 1093-1103.
de Ruyter,P.G., Kuipers,O.P., and de Vos,W.M. (1996) Controlled gene expression systems for Lactococcus lactis with the food-grade inducer nisin Appl.Environ.Microbiol. 62: 3662-3667.
Sambrook,J., Fritsch,E.F., and Maniatis,T. (1989) Molecular cloning: a laboratory manual Cold Spring Harbor, N.Y.: Cold Spring Harbor laboratory Press.
Sanders,J.W., Venema,G., Kok,J., and Leenhouts,K. (1998) Identification of a sodium chloride-regulated promoter in Lactococcus lactis by single-copy chromosomal fusion with a reporter gene Mol. Gen. Genet. 257: 681-685.
Sanz,Y., Lanfermeijer,F.C., Renault,P., Bolotin,A., Konings,W.N., and Poolman,B. (2001) Genetic and functional characterization of dpp genes encoding a dipeptide transport system in Lactococcus lactis Arch.Microbiol. 175: 334-343.
Serror,P., Sonenshein,A.L. (1996a) CodY is required for nutritional repression of Bacillus subtilis genetic competence J.Bacteriol. 178: 5910-5915.
Serror,P., Sonenshein,A.L. (1996b) Interaction of CodY, a novel Bacillus subtilis DNA-binding protein, with the dpp promoter region Mol.Microbiol. 20: 843-852.
Spee,J.H., de Vos,W.M., and Kuipers,O.P. (1993) Efficient random mutagenesis method with adjustable mutation frequency by use of PCR and dITP Nucleic Acids Res. 21: 777-778.
Tynkkynen,S., Buist,G., Kunji,E., Kok,J., Poolman,B., Venema,G., and Haandrikman,A. (1993) Genetic and biochemical characterization of the oligopeptide transport system of Lactococcus lactis J.Bacteriol. 175: 7523-7532.
van Asseldonk, M., A. Simons, H. Visser, W. M. de Vos, and G. Simons. 1993. Cloning, nucleotide sequence, and regulatory analysis of the Lactococcus lactis dnaJ gene. J.Bacteriol. 175:1637-1644.
Visser, S., Exterkate, F. A., Slangen, K. J. and De Veer, G. J. C. M. (1986). Comparative study of the action of cell wall proteinases from various strains of Streptococcus cremoris on bovine as1- b- and k-casein. Appl.Environ.Microbiol. 52, 1162-1166.
de Vos, W. M., Vos, P., de Haard, H. and Boerrigter, I. (1989). Cloning and expression of the Lactococcus lactis subsp. cremoris SK11 gene encoding an extracellular serine proteinase. Gene 85, 169-176.

## Claims

1. A method for regulating the expression of a gene of interest in a host cell that comprises a CodY protein comprising providing said cell with a gene of interest in operable linkage with a promoter and at least one CodY target sequence, wherein
- the number of CodY target sequences of said promoter is increased, resulting in more than one CodY target sequences, and wherein
- said at least one CodY target sequence comprises the sequence WHAATTDTCWGAHAAWTNNRWNADWW or AWTTDTCAGAAWWWT or AATTTTCWGAAAATT or a sequence as depicted in Table 4, Table 4A, Table 5, Table 6, Table 7 and/or Table 8 and wherein W = T or A, R = A or G, D = not C and H = not G.

2. A method according to claim 1, wherein said CodY target sequence is heterologous with regard to said promoter.

3. A method according to any one of claims 1 to 2, wherein said gene of interest is a gene from a gram-positive bacterium.

4. A method according to any one of claims 1 to 3, wherein said gene of interest encodes a protease or a peptidase or an anti-microbial peptide or a vitamin.

5. A method according to any one of claims 1-4, wherein said CodY target sequence comprises an ATGTTCA sequence and an inversely repeated ATGTTCA sequence.

6. A method according to claim 5, wherein said nucleic acid sequence comprises a spacing of about 9 base pairs between said ATGTTCA sequence and said inversely repeated ATGTTCA sequence.

7. A method according to claim 5 or 6, wherein said nucleic acid sequence comprises the sequence ATGTTCAGAAAATTCATGAACAT.

8. A method according to any one of claims 1 to 7, further comprising influencing the binding between said CodY protein and said at least one CodY target sequence.

9. A method according to claim 8, wherein said binding is regulated by subjecting said cell to a change in a growth condition.

10. A method according to claim 8 or 9, wherein said binding is regulated by subjecting said cell to a growth limiting condition.

11. A method according to claim 10, wherein said growth limiting condition is a limited availability of a nitrogen source.

12. A method according to any one of claims 1 to 11, wherein said host cell is a cell from a (dairy) food production species.

13. A method according to claim 12, wherein said species is selected from a *Lactococcus* or *Lactobacillus* or *Streptococcus* or *Leuconostoc* or *Pediococcus* or *Bifidobacterium* or *Carnobacterium* or *Propionibacterium.*

14. A method according to any one of claims 1 to 13, wherein said host cell is provided with a nucleic acid encoding a CodY protein.

## Patentansprüche

1. Verfahren zur Regulation der Expression eines Gens von Interesse in einer Wirtszelle, umfassend ein CodY-Protein, umfassend die Bereitstellung der Zelle mit einem Gen von Interesse in operabler Verbindung mit einem Promotor und mindestens einer CodY-Zielsequenz, wobei
- die Zahl der CodY-Zielsequenzen des Promotors erhöht ist, was zu mehr als einer CodY-Zielsequenz führt, und wobei
- die mindestens eine CodY-Zielsequenz die Sequenz WHAATTDTCWGAHAAWTNNRWNADWW oder AWTTDTCAGAAWWWT oder AATTTTCWGAAAATT oder eine Sequenz, wie in Tabelle 4, Tabelle 4A, Tabelle 5, Tabelle 6, Tabelle 7 und/oder Tabelle 8 dargestellt, umfasst und wobei W = T oder A, R = A oder G, D = nicht C und H = nicht G ist.

2. Verfahren gemäss Anspruch 1, wobei die CodY-Zielsequenz in bezug auf den Promotor heterolog ist.

3. Verfahren gemäss einem der Ansprüche 1 und 2, wobei das Gen von Interesse ein Gen eines gram-positiven Bakteriums ist.

4. Verfahren gemäss einem der Ansprüche 1 bis 3, wobei das Gen von Interesse eine Protease oder eine Peptidase oder ein antimikrobielles Peptid oder ein Vitamin codiert.

5. Verfahren gemäss einem der Ansprüche 1 bis 4, wobei die CodY-Zielsequenz eine ATGTTCA-Sequenz und eine invers wiederholte ATGTTCA-Sequenz umfasst.

6. Verfahren gemäss Anspruch 5, wobei die Nukleinsäuresequenz eine Beabstandung von ungefähr 9 Basenpaaren zwischen der ATGTTCA-Sequenz und der invers wiederholten ATGTTCA-Sequenz umfasst.

7. Verfahren gemäss Anspruch 5 oder 6, wobei die Nukleinsäuresequenz die Sequenz ATGTTCAGAAAATTCATGAACAT umfasst.

8. Verfahren gemäss einem der Ansprüche 1 bis 7, weiterhin umfassend die Beeinflussung der Bindung zwischen dem CodY-Protein und der mindestens einen CodY-Zielsequenz.

9. Verfahren gemäss Anspruch 8, wobei die Bindung **dadurch** reguliert wird, dass die Zelle einer Veränderung hinsichtlich einer Wachstumsbedingung unterworfen wird.

10. Verfahren gemäss Anspruch 8 oder 9, wobei die Bindung **dadurch** reguliert wird, dass die Zelle einer das Wachstum begrenzenden Bedingung unterworfen wird.

11. Verfahren gemäss Anspruch 10, wobei die das Wachstum begrenzende Bedingung eine begrenzte Verfügbarkeit einer Stickstoffquelle ist.

12. Verfahren gemäss einem der Ansprüche 1 bis 11, wobei die Wirtszelle eine Zelle einer (milchprodukt)-Nahrungsmittelproduktionsart ist.

13. Verfahren gemäss Anspruch 12, wobei die Art gewählt ist aus Lactococcus oder Lactobacillus oder Streptococcus oder Leuconostoc oder Pediococcus oder Bifidobacterium oder Cornobacterium oder Propionibacterium.

14. Verfahren gemäss einem der Ansprüche 1 bis 13, wobei die Wirtszelle mit einer Nukleinsäure, die ein CodY-Protein codiert, versehen wird.

## Revendications

1. Procédé de régulation de l'expression d'un gène d'intérêt dans une cellule hôte qui comprend une protéine CodY consistant à fournir à ladite cellule un gène d'intérêt en liaison fonctionnelle avec un promoteur et au moins une séquence cible CodY, où
le nombre de séquences cible CodY dudit promoteur est augmenté, ayant pour résultat plus d'une séquence cible CodY, et où
ladite au moins une séquence cible CodY comprend la séquence WHAATTDTCWGAHAAWTNNRWNADWW ou AWTTDTCAGAAWWWT ou AATTTTCWGAAAATT ou une séquence comme décrit dans le tableau 4, le tableau 4A, le tableau 5, le tableau 6, le tableau 7 et/ou le tableau 8 et où W = T ou A, R = A ou G, D non = C et H non = G.

2. Procédé selon la revendication 1, où ladite séquence cible CodY est hétérologue par rapport audit promoteur.

3. Procédé selon l'une quelconque des revendications 1 à 2, où ledit gène d'intérêt est un gène provenant d'une bactérie Gram positive.

4. Procédé selon l'une quelconque des revendications 1 à 3, où ledit gène d'intérêt code pour une protéase ou une peptidase ou un peptide antimicrobien ou une vitamine.

5. Procédé selon l'une quelconque des revendications 1 à 4, où ladite séquence cible CodY comprend une séquence ATGTTCA et une séquence ATGTTCA répétée inversement.

6. Procédé selon la revendication 5, où ladite séquence d'acide nucléique comprend un espacement d'environ 9 paires de bases entre ladite séquence ATGTTCA ladite séquence ATGTTCA répétée inversement.

7. Procédé selon la revendication 5 ou 6, où ladite séquence d'acide nucléique comprend la séquence ATGTTCAGAAAATTCATGAACAT.

8. Procédé selon l'une quelconque des revendications 1 à 7, consistant en outre à influencer la liaison entre ladite protéine CodY et ladite au moins une séquence cible CodY.

9. Procédé selon la revendication 8, où ladite liaison est régulée en soumettant ladite cellule à une modification de condition de croissance.

10. Procédé selon la revendication 8 ou 9, où ladite liaison est régulée en soumettant ladite cellule à une condition de limitation de croissance.

11. Procédé selon la revendication 10, où ladite condition de limitation de croissance est une disponibilité limitée d'une source d'azote.

12. Procédé selon l'une quelconque des revendications 1 à 11, où ladite cellule hôte est une cellule provenant d'une espèce de production alimentaire (laitière).

13. Procédé selon la revendication 12, où ladite espèce est choisie parmi le *Lactococcus* ou *Lactobacillus* ou *Streptococcus* ou *Leuconostoc* ou *Pediococcus* ou *Bifidobacterium* ou *Carnobacterium* ou *Propionibacterium.*

14. Procédé selon l'une quelconque des revendications 1 à 13, où ladite cellule hôte est munie d'un acide nucléique codant pour une protéine CodY.
